(19) Europäisches Patentamt
European Patent Office
Office européen des brevets

(11) EP 1 399 116 B1

(12) **FASCICULE DE BREVET EUROPEEN**

(45) Date de publication et mention de la délivrance du brevet:
**05.08.2009 Bulletin 2009/32**

(21) Numéro de dépôt: **02747517.7**

(22) Date de dépôt: **07.06.2002**

(51) Int Cl.:
***A61Q 5/06*** (2006.01) ***A61Q 5/10*** (2006.01)
***A61K 8/19*** (2006.01) ***A61K 8/30*** (2006.01)
***A61K 8/72*** (2006.01)

(86) Numéro de dépôt international:
**PCT/FR2002/001963**

(87) Numéro de publication internationale:
**WO 2002/100365 (19.12.2002 Gazette 2002/51)**

(54) **UTILISATION DE COMPOSES DICATIONIQUES POUR LA TEINTURE DES FIBRES KERATINIQUES HUMAINES ET COMPOSITIONS LES CONTENANT**

VERWENDUNG DIKATIONISCHER VERBINDUNGEN ZUR FÄRBUNG KERATINISCHER FASERN UND DIESE ENTHALTENDE ZUSAMMENSETZUNGEN

USE OF DICATIONIC COMPOUNDS FOR DYEING HUMAN KERATINOUS FIBRES AND COMPOSITIONS CONTAINING SAME

(84) Etats contractants désignés:
**AT BE CH CY DE DK ES FI FR GB GR IE IT LI LU MC NL PT SE TR**

(30) Priorité: **12.06.2001 FR 0107683**

(43) Date de publication de la demande:
**24.03.2004 Bulletin 2004/13**

(73) Titulaire: **L'ORÉAL**
**75008 Paris (FR)**

(72) Inventeurs:
- **PLOS, Grégory**
  **F-75011 Paris (FR)**
- **SAMAIN, Henri**
  **F-91570 Bievres (FR)**

(74) Mandataire: **Wattremez, Catherine**
**L'OREAL - D.I.P.I.**
**25-29 Quai Aulagnier**
**92600 Asnières (FR)**

(56) Documents cités:
**EP-A- 0 318 294**
**EP-A- 0 696 619**
**EP-A- 1 022 016**
**EP-A- 1 133 975**
**WO-A-02/31056**
**DE-A- 4 128 490**
**DE-A- 19 802 940**
**US-A- 3 578 386**
**US-A- 5 708 151**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 399 116 B1

## Description

L'invention concerne l'utilisation de composés dicationiques, à titre de colorants directs dans une composition tinctoriale pour la teinture des fibres kératiniques humaines et en particulier des cheveux. L'invention concerne également les compositions de teinture directe et éclaircissante les contenant ainsi que les procédés de teinture correspondants.

On a déjà décrit et proposé certains colorants dicationiques pour colorer du papier; des azo- ou di-azoimidazoles cationiques ont ainsi été décrits dans les brevets américains N°- 5 708 151 et 5 674 299.

Les colorants cationiques décrits dans la présente invention n'ont jamais été proposés pour être utilisés en cosmétique.

Or, dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration directe éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.

Le deuxième est la.coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation. La variété des molécules mises en jeu, qui sont constituées d'une part, par les bases d'oxydation et d'autre part, par les coupleurs, permet l'obtention d'une palette très riche en coloris.

Ainsi la coloration capillaire, qu'elle soit directe ou d'oxydation, est réalisée classiquement avec des molécules de faible poids moléculaire. En effet, un faible poids moléculaire permet, dans le cas de la coloration directe, une libre diffusion des colorants à l'intérieur du cheveu, et dans le cas de la coloration d'oxydation, de laisser diffuser les espèces réactives à l'intérieur de la fibre avant la réaction d'oxydation.

Et généralement, on a jusqu'ici évite d'utiliser des molécules de poids moléculaire important car ces molécules présentent l'inconvénient d'être sélectives, c'est à dire qu'elles conduisent à des écarts de coloration tout au long d'une même fibre kératinique.

Or, en teinture capillaire, les colorants doivent être les moins sélectifs possible, c'est à dire permettre d'obtenir des écarts de coloration les plus faibles possible tout au long d'une même fibre kératinique qui peut être en effet différemment sensibilisée (i.e. abîmée) entre sa pointe et sa racine.
Ce problème de sélectivité rend donc les colorants de poids moléculaire élevé généralement inutilisables.

Voici maintenant qu'après d'importantes recherches menées sur la question, la Demanderesse vient de découvrir qu'il est possible d'obtenir des compositions de teinture directe et directe éclaircissante avec des colorants qui permettent d'obtenir des nuances avec de faibles sélectivités si les dits colorants sont choisis parmi ceux de formules (I), (II), ou (III) décrites ci-après.
Les nuances obtenues avec lesdits colorants sont par ailleurs puissantes chromatiques (lumineuses) et résistent bien aux diverses agressions que peuvent subir les cheveux.

Ces découvertes sont à la base de la présente invention.

La présente invention a ainsi pour premier objet, l'utilisation pour la teinture des fibres kératiniques humaines et plus particulièrement des cheveux, à titre de colorants directs, de composés dicationiques de formules (I), (II), ou (III) décrites ci-après, dans une composition cosmétique.

Elle a pour second objet, une composition de teinture directe pour fibres kératiniques humaines, et en particulier les cheveux, comprenant, dans un milieu approprié pour la teinture, une quantité efficace d'au moins un colorant direct dicationique de formules (I), (II), ou (III), un ou plusieurs tensioactifs, indifféremment choisis, seuls ou en mélange, parmi les tensioactifs anioniques, non ioniques, amphotères, zwittérioniques et cationiques.

Un autre objet de l'invention porte sur une composition prête à l'emploi pour la teinture des fibres kératiniques humaines, et en particulier des cheveux, qui contient au moins un colorant direct dicationique de formules (I), (II), ou (III) et un agent oxydant. Par "composition prête à l'emploi", on entend, au sens de l'invention, la composition destinée à être appliquée telle quelle sur les fibres kératiniques, c'est à dire qu'elle peut être stockée telle quelle avant utilisation ou résulter du mélange extemporané de deux ou plusieurs compositions.

L'invention vise également un procédé de teinture directe des fibres kératiniques humaines et en particulier

des cheveux, consistant à appliquer sur les fibres une composition contenant, dans un milieu approprié pour la teinture, au moins un colorant direct dicationique de formules (I), (II), ou (III).

L'invention vise aussi un procédé de teinture directe éclaircissante des fibres kératiniques humaines et en particulier des cheveux, consistant à appliquer sur les fibres un mélange extemporané d'une composition contenant, dans un milieu approprié pour la teinture, au moins un colorant direct dicationique de formules (I), (II), ou (III) et d'une composition contenant au moins un agent oxydant.

L'invention a également pour objet un dispositif pour la teinture directe éclaircissante des fibres kératiniques humaines et en particulier des cheveux, ou "kit" de teinture, qui comprend un premier compartiment renfermant, dans un milieu approprié pour la teinture, au moins un colorant direct dicationique de formules (I), (II), ou (III) et un deuxième compartiment renfermant un agent oxydant.

Ce dispositif peut être équipé d'un moyen permettant de délivrer sur les cheveux le mélange souhaité, tels que les dispositifs décrits dans le brevet FR-2 586 913 au nom de la demanderesse.

Mais d'autres caractéristiques, aspects, objets et avantages de l'invention apparaîtront encore plus clairement à la lecture de la description et des exemples qui suivent.

Colorant direct dicationique

Les colorants directs dicationiques selon la présente invention sont choisis parmi ceux de formules (I), (II), ou (III) suivantes :

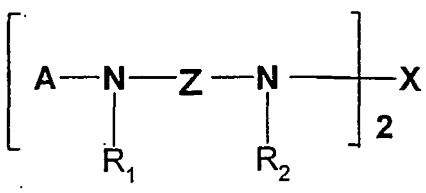

(I)

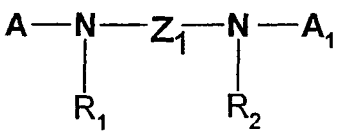

(II)

formules (I) où (II) dans lesquelles :

- A et A1, indépendamment l'un de l'autre désignent un radical de formule (a) suivante

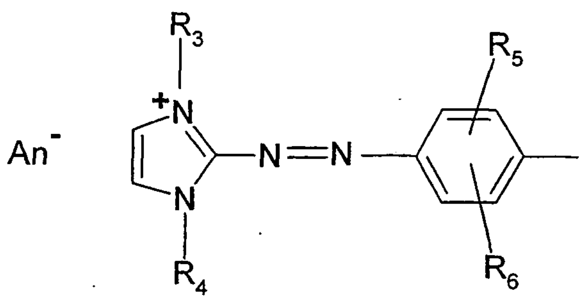

(a)

- Z désigne un radical aliphatique ou aromatique,
- $Z_1$ désigne un radical alkyle,
- $R_1$ et $R_2$, indépendamment l'un de l'autre, désignent un atome d'hydrogène, ou un radical $(C_1-C_4)$alkyl, ou $(C_1-C_4)$ afkyl substitué par un ou plusieurs atomes d'halogène, un radical hydroxyl, carboxyl, cyano, un radical $(C_1-C_4)$ alcoxy, un radical $(C_1-C_4)$alcoxy substitué par un ou plusieurs radicaux hydroxyl, ou $(C_1-C_4)$alcoxy, un radical amino,

alkylamino, dialkylamino, aminocarbonyl, phényl, phénoxy ou phénylaminocarbonyl, dans lequel le radical phényl est non substitué ou substitué par un radical $(C_1-C_4)$alkyl, $(C_1-C_4)$alcoxy ou phénoxy,

ou encore $R_1$ et $R_2$ forment ensemble, avec les deux atomes d'azote qui les portent et le radical Z, un cycle pipérazinique,

- X est un radical de pontage choisi parmi : -CO- ; $-CO-CH_2-CH_2-CO-$ ; -CO-CO- ; 1,4-dicarbonylphényl ; $-CH_2-CH_2-$ ; ou une triazine de formules (b) ou (c) suivantes:

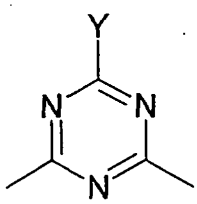

**(b)**

ou

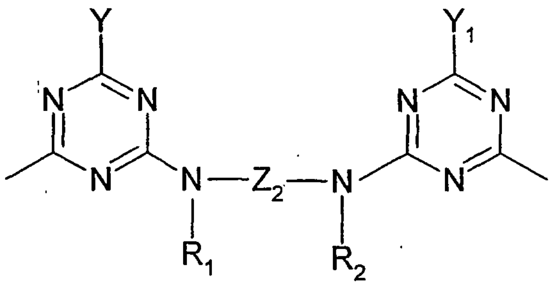

**(c)**

dans lesquelles :

Y et Y1, indépendamment l'un de l'autre désignent un atome d'halogène, ou un radical hydroxyl, ou amino, ou monoalkylamino, ou dialkylamino, ou 1-pipéridino, ou morpholino, ou 1-pipérazino, le radical pipérazino étant non substitué, ou substitué sur l'atome d'azote non attaché au cycle triazine par un radical $(C_1-C_4)$alkyl, lesdits radicaux alkyls étant non substitués ou substitués par hydroxyl, amino, mono-$(C_1-C_4)$alkylamino ou di-$(C_1-C_4)$alkylamino,

- $Z_2$ désigne un radical $(C_2-C_8)$alkylène ou forme, avec les deux atomes d'azote adjacents et les radicaux $R_1$ et $R_2$, un cycle pipérazinique,
- dans le radical de formule (a),
- $R_3$ et $R_4$, indépendamment l'un de l'autre, désignent un atome d'hydrogène, ou un radical $(C_1-C_4)$alkyl, ou $(C_1-C_4)$ alkyl substitué par un ou plusieurs atomes d'halogène, un radical hydroxyl, carboxyl, cyano, un radical $(C_1-C_4)$ alcoxy, un radical $(C_1-C_4)$alcoxy substitué par un radical hydroxyl ou $(C_1-C_4)$alcoxy, un radical amino, alkylamino, dialkylamino, aminocarbonyl, phényl, phénoxy ou phénylaminocarbonyl, dans lequel le radical phényl est non substitué ou substitué par un radical $(C_1-C_4)$alkyl, $(C_1-C_4)$alcoxy ou phénoxy,
- $R_5$ et $R_6$, indépendamment l'un de l'autre, désignent un atome d'hydrogène, un radical $(C_1-C_4)$alkyl ou $(C_1-C_4)$ alcoxy éventuellement substitués par un radical hydroxyl, carboxyl, halogène, cyano, $(C_1-C_4)$alcoxy éventuellement substitué par un radical hydroxyl ou $(C_1-C_4)$alcoxy, un radical amino, alkylamino, dialkylamino, aminocarbonyl, phényl, phénoxy ou phénylaminocarbonyl, dans lequel le radical phényl est non substitué ou substitué par un radical $(C_1-C_4)$alkyl, $(C_1-C_4)$alcoxy ou phénoxy, $-An^-$ désigne un anion.

De préférence, selon l'invention, dans la formule (I),

- $R_1$ et $R_2$, indépendamment l'un de l'autre, désignent hydrogène, $(C_1-C_4)$alkyl substitué par hydroxyl ou $(C_1-C_4)$ alcoxy et plus particulièrement encore désignent hydrogène ou méthyle,
- Z désigne un radical alkyle en $C_2-C_8$ linéaire ou ramifié ou cyclique, éventuellement substitué par un hydroxy, alcoxy, halogène, la chaîne dudit radical étant éventuellement interrompue par un groupe -O- ou $-NR_1-$ ; un radical 1,4-phényl, un radical 1,4-naphtyl éventuellement substitué par un alkyle, alcoxy, halogène ; Z pouvant formé avec $R_1$, $R_2$ et les 2 atomes d'azote, un cycle pipérazine, Z désigne préférentiellement un radical phényl non substitué, un radical phényl ou naphtyl substitué par 1 ou 2 radicaux méthyl ou méthoxy, un radical pipérazine par liaison avec

$R_1$, $R_2$ et les 2 atomes d'azote, un radical ($C_2$-$C_4$)alkylène non subsitué ou substitué par un ou 2 hydroxyl,
- X désigne un groupement de formule (b).

De préférence, selon l'invention, dans la formule (II),

- Z1 désigne un radical alkyle en $C_2$-$C_8$ linéaire ou ramifié ou cyclique, éventuellement substitué par un hydroxy, alcoxy, halogène, la chaîne dudit radical étant éventuellement interrompue par un groupe -O- ou -$NR_1$- ; un cycle pipérazine formé avec $R_1$, $R_2$ et les deux atomes d'azote, $Z_1$ désigne préférentiellement un radical ($C_2$-$C_6$)alkylène non substitué ou substitué par un ou plusieurs hydroxyl, un cycle pipérazine formé avec $R_1$, $R_2$ et les deux atomes d'azote ; et plus particulièrement encore un radical ($C_2$-$C_4$)alkylène non substitué,
- $R_3$ et $R_4$ désignent méthyl ou éthyl, et $R_5$ et $R_6$ désignent hydrogène, méthyl, ou méthoxy.

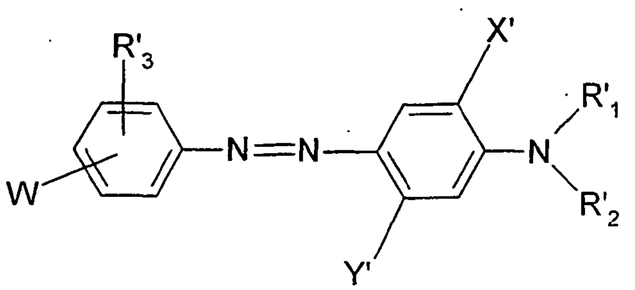

**(III)**

formule (III) dans laquelle,
- le nombre de charges cationiques est de deux,
- X' et Y', indépendamment l'un de l'autre, désignent hydrogène, halogène, ($C_1$-$C_4$)alkyl, ($C_1$-$C_4$)alcoxy, ($C_1$-$C_4$) alkylcarbonylamino, arylcarbonylamino, uréido, ou aryluréido,
- $R'_1$ désigne hydrogène, un radical alkyl ou aryl substitués, un radical alkyl ou aryl non substitués, ou la même désignation que $R'_2$
- $R'_2$ est un radical de formule (d) suivante:

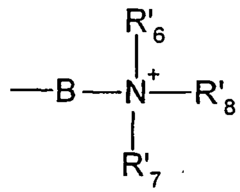

**(d)**

dans laquelle :

- B désigne un radical alkylène linéaire ou ramifié,
- $R'_6$ désigne hydrogène ou alkyl substitué ou non substitué,
- $R'_7$ et $R'_8$, indépendamment l'un de l'autre désignent alkyl substitué ou non substitué,
- $R'_6$ et $R'_7$, ensemble avec l'azote, forment un cycle à 5, 6, ou 7 chaînons, substitué ou non substitué, pouvant contenir d'autres hétéroatomes, ou bien $R'_6$ et $R'_7$ et $R'_8$ forment ensemble un cycle pyridinium,
- $R'_3$ désigne hydrogène, halogène, ($C_1$-$C_4$)alkyl, ($C_1$-$C_4$)alcoxy,
- W est un radical de formule (e) suivante :

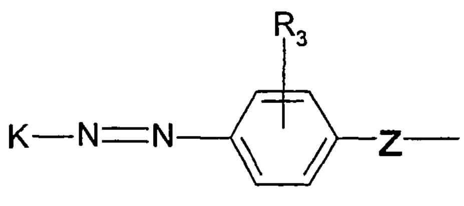

**(e)**

dans laquelle:

- K est un radical de couplage,
- Z désigne un radical de pontage choisi parmi les radicaux de formules :

$-NR'_9-CO-;$

$-CO-NR'_9-NR'_9-CO-;$

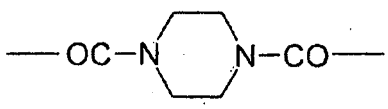

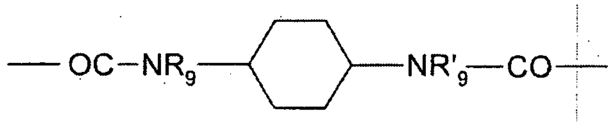

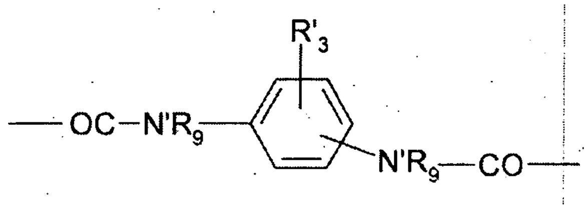

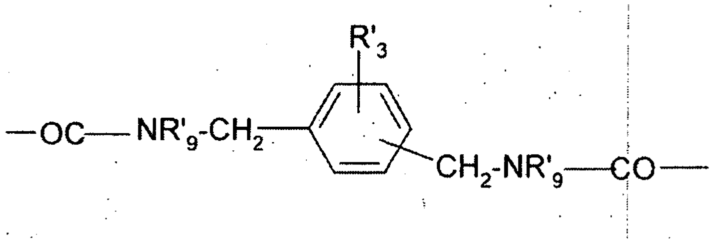

ou bien

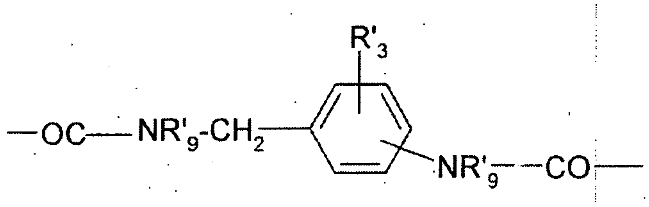

et dans lesquels $R'_9$ désigne, hydrogène, $(C_2-C_4)$alkylène non substitué ou substitué, le radical alkylène étant linéaire ou ramifié et pouvant être interrompu par un ou plusieurs groupements choisis parmi : -NR'9-, -O-, -S-.

De préférence, selon l'invention, dans la formule (III),

B désigne éthylène, n-propylène, isopropylène ou n-butylène,
K désigne un composé de couplage choisi parmi ceux de formule (f), (g) ou (h) suivantes

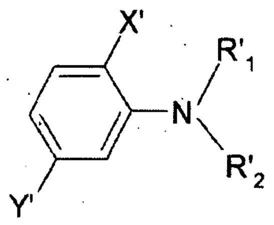

(f)

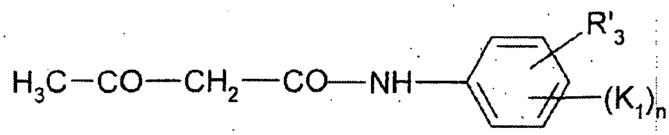

(g)

ou

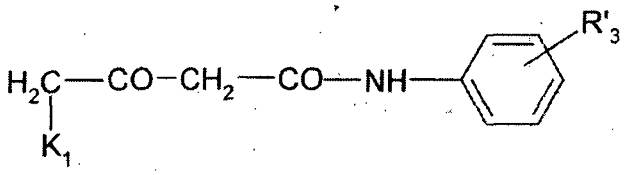

(h)

dans lesquelles,

- X', Y' et $R'_1$, $R'_2$ et $R'_3$, ont la même désignation que dans la formule (III),
- n est égal à 1 ou 2,
- $K_1$ désigne le radical de formule:

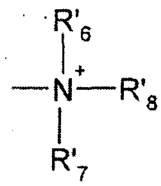

Selon la présente invention, parmi les composés de formule (I) ou de formule (II), on peut citer plus particulièrement le composé commercialisé par la société CIBA sous la dénomination PERGASOL VIOLET F-R de formule suivante :

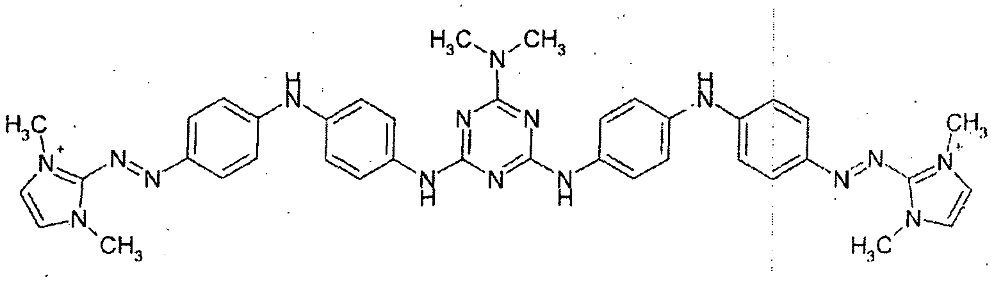

(I)1

On peut également citer les composés de formule (I) répondant aux formules (I)2 à (I)7 suivantes :

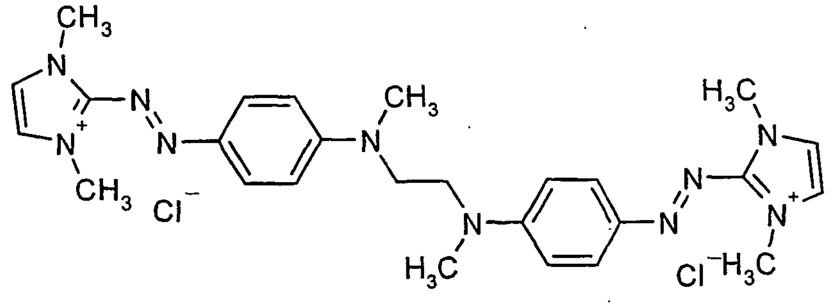

(I)2

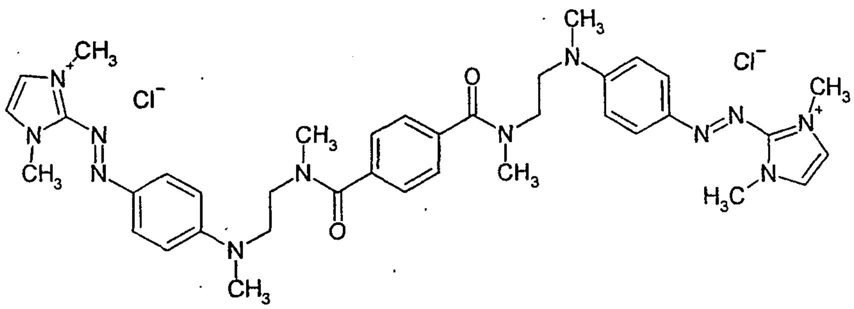

(I)3

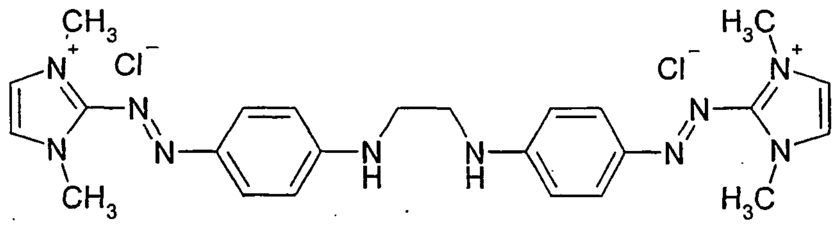

(I)4

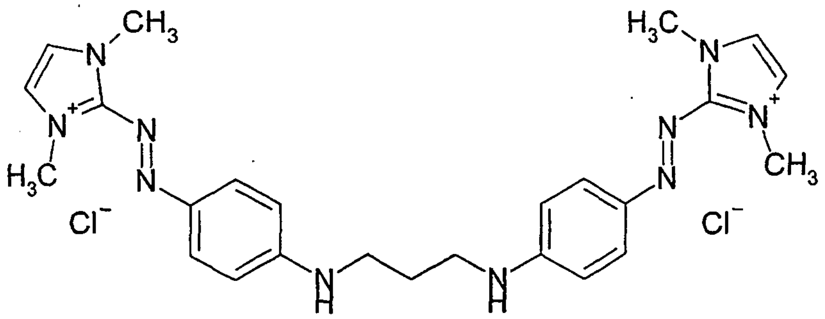

(I)5

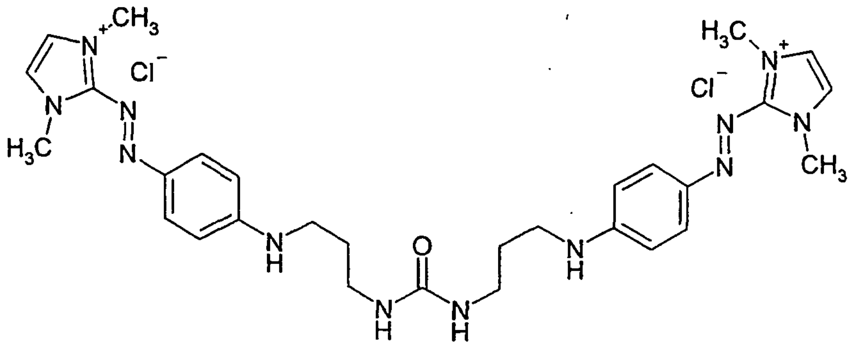

(I)6

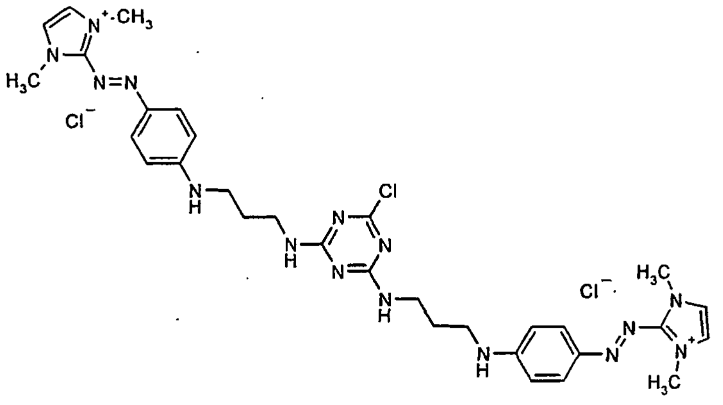

(I)7

Selon la présente invention, parmi les composés de formule (III), on peut citer plus particulièrement le composé commercialisé par la société CIBA sous la dénomination PERGASOL ORANGE F-3G de formule suivante :

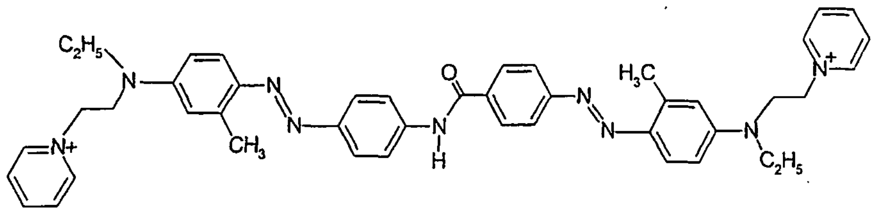

(I)8

Les colorants de formule (I), (II), ou (III) selon la présente invention sont connus en tant que tels, et décrits et préparés dans les brevets US- 5 674 299 ou US- 5 708 151, dont le contenu fait partie intégrante de la présente invention.
Ils sont généralement présents dans la composition de teinture de la présente invention dans des proportions allant d'environ 0,01 à 40%, de préférence d' environ 0,1 à 20% en poids, par rapport au poids total de la composition.
Le milieu de la composition approprié pour la teinture, est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations allant d'environ 0,5 à 20% et, de préférence d'environ 2 à 10% en poids par rapport au poids total de la composition.
La composition de teinture peut encore contenir une quantité efficace d'autres agents, par ailleurs antérieurement connus en coloration directe, tels que divers adjuvants usuels comme des agents tensio-actifs bien connus de l'état de la technique et de type anionique, cationique, non-ionique, amphotère, zwittérionique ou leurs mélanges, des agents épaississants, des agents anti-oxydants, des parfums, des agents dispersants, des agents de conditionnement dont notamment dés polymères cationiques ou amphotères, des agents opacifiants, des agents séquestrants tel que l'EDTA et l'acide étidronique, des filtres UV, des cires, des silicones volatiles ou non, cycliques ou linéaires ou ramifiées, organomodifiées (notamment par des groupements amines) ou non, des agents conservateurs, des céramides, des pseudocéramides, des huiles végétales, minérales ou de synthèse, les vitamines ou provitamines comme le panthénol, des polymères associatifs non-ioniques, anioniques, amphotères ou cationiques.

Polymères associatifs

Les polymères associatifs sont des polymères hydrosolubles capables, dans un milieu aqueux, de *s'associer* réversiblement entre eux ou avec d'autres molécules.
Leur structure chimique comprend des zones hydrophiles, et des zones hydrophobes caractérisées par au moins une chaîne grasse.

Polymères associatifs de type anionique :

On peut citer parmi eux :

- **(I)** ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

$$CH_2 = C\ R'\ CH_2\ O\ B_n\ R \qquad (I)$$

dans laquelle R' désigne H ou $CH_3$, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl ($C_{18}$).

Des polymères associatifs anioniques de ce type sont décrits et préparés, selon un procédé de polymérisation en émulsion, dans le brevet EP-0 216 479.

Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1 % en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).

- **(II)** ceux comportant au moins un motif hydrophile de type acide carboxylique insaturé oléfinique, et au moins un motif hydrophobe de type ester d'alkyl ($C_{10}$-$C_{30}$) d'acide carboxylique insaturé.

De préférence, ces polymères sont choisis parmi ceux dont le motif hydrophile de type acide carboxylique insaturé oléfinique correspond au monomère de formule (II) suivante :

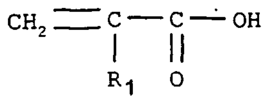

**(II)**

dans laquelle, $R_1$ désigne H ou $CH_3$ ou $C_2H_5$, c'est-à-dire des motifs acide acrylique, acide méthacrylique ou acide éthacrylique, et dont le motif hydrophobe de type ester d'alkyl ($C_{10}$-$C_{30}$) d'acide carboxylique insaturé correspond au monomère de formule (III) suivante

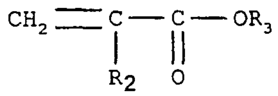

**(III)**

dans laquelle, $R_2$ désigne H ou $CH_3$ ou $C_2H_5$ (c'est-à-dire des motifs acrylates, méthacrylates ou éthacrylates) et de préférence H (motifs acrylates) ou $CH_3$ (motifs méthacrylates), $R_3$ désignant un radical alkyle en $C_{10}$-$C_{30}$, et de préférence en $C_{12}$-$C_{22}$.

Des esters d'alkyles ($C_{10}$-$C_{30}$) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle. Des polymères anioniques de ce type sont par exemple décrits et préparés, selon les brevets US-3 915 921 et 4 509 949.

Parmi ce type de polymères associatifs anioniques, on utilisera plus particulièrement des polymères formés à partir d'un mélange de monomères comprenant :

(i) essentiellement de l'acide acrylique,

(ii) un ester de formule (III) décrite ci-dessus et dans laquelle $R_2$ désigne H ou $CH_3$, $R_3$ désignant un radical alkyle ayant de 12 à 22 atomes de carbone,

(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on utilisera plus particulièrement ceux constitués de 95 à 60% en poids d'acide acrylique (motif hydrophile), 4 à 40% en poids d'acrylate d'alkyles en $C_{10}$-$C_{30}$ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en $C_{10}$-$C_{30}$ (motif hydrophobe), et 0,1 à 0,6% en poids de monomère polymérisable réticulant tel que ceux décrits précedemment.

Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.

- **(III)** les terpolymères d'anhydride maléique/α-oléfine en $C_{30}$-$C_{38}$/ maléate d'alkyle tel que le produit (copolymère anhydride maléique/α-oléfine en $C_{30}$-$C_{38}$/maléate d'isopropyle) vendu sous le nom PERFORMA V 1608 par la société NEWPHASE TECHNOLOGIES.

- **(IV)** les terpolymères acryliques comprenant :

  (a) environ 20% à 70% en poids d'un acide carboxylique à insaturation α,β-monoéthylénique,
  (b) environ 20 à 80% en poids d'un monomère à insaturation α,β-monoéthylénique non-tensio-actif différent de (a),
  (c) environ 0,5 à 60% en poids d'un mono-uréthane non-ionique qui est le produit de réaction d'un tensio-actif monohydrique avec un monoisocyanate à insaturation monoéthylénique,

  tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.

- **(V)** les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C1C4.
A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Polymères associatifs de type cationique

Selon la présente invention, ils sont choisis de préférence parmi les dérivés de cellulose quaternisée et les polyacrylates à groupements latéraux aminés non cycliques.
Les dérivés de cellulose quaternisée sont, en particulier,

- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
On peut indiquer comme exemples d'alkylhydroxyéthyl-celluloses quaternisées à chaînes grasses en $C_8$-$C_{30}$, les produits QUATRISOFT LM 200, QUATRISOFT LM-X 529-18-A, QUATRISOFT LM-X 529-18B (alkyle en $C_{12}$) et QUATRISOFT LM-X 529-8 (alkyle en $C_{18}$) commercialisés par la société AMERCHOL et les produits CRODACEL QM, CRODACEL QL (alkyle en $C_{12}$) et CRODACEL QS (alkyle en C18) commercialisés par la société CRODA.

Polymères associatifs amphotères

Ils sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.
Les polymères associatifs amphotères préférés selon l'invention comprennent, ou sont préparés en copolymérisant :

1) au moins un monomère de formule (Ia) ou (Ib):

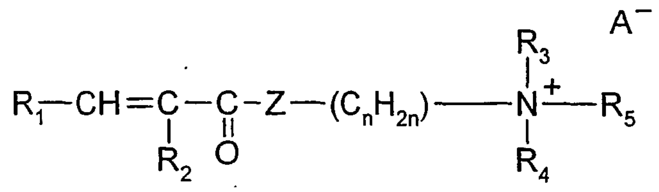

(Ia)

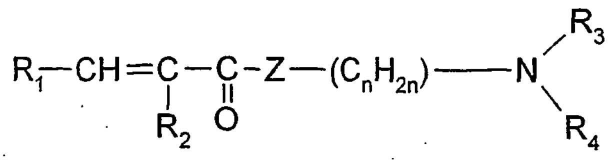

(Ib)

dans lesquelles, $R_1$ et $R_2$, identiques ou différents, représentent un atome d'hydrogène ou un radical méthyle, $R_3$, $R_4$ et $R_5$, identiques ou différents, représente un radical alkyle linéaire ou ramifié ayant de 1 à 30 atomes de carbone,

Z représente un groupe NH ou un atome d'oxygène,
n est un nombre entier de 2 à 5,
A- est un anion issu d'un acide organique ou minéral, tel qu'un anion méthosulfate ou un halogénure tel que chlorure ou bromure;

2) au moins un monomère de formule (II)

$$R_6\text{-}CH = CR_7\text{-}COOH \qquad (II)$$

dans laquelle, $R_6$ et $R_7$, identiques ou différents, représentent un atome d'hydrogène ou un radical méthyle; et

3) au moins un monomère de formule (III) :

$$R_6\text{-}CH= CR_7\text{-}COXR_8 \qquad (III)$$

dans laquelle $R_6$ et $R_7$, identiques ou différents, représentent un atome d'hydrogène ou un radical méthyle, X désigne un atome d'oxygène ou d'azote et $R_8$ désigne un radical alkyle linéaire ou ramifié ayant de 1 à 30 atomes de carbone ; l'un au moins des monomères de formule (Ia), (Ib) ou (III) comportant au moins une chaîne grasse.

Les monomères de formule (Ia) et (Ib) de la présente invention sont choisis, de préférence, dans le groupe constitué par :

- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide,

ces monomères étant éventuellement quaternisés, par exemple par un halogénure d'alkyle en $C_1$-$C_4$ ou un sulfate de dialkyle en $C_1$-$C_4$.

Plus particulièrement, le monomère de formule (Ia) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué

par des acrylates ou méthacrylates d'alkyle en $C_{12}$-$C_{22}$ et plus particulièrement en $C_{16}$-$C_{18}$.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 moles% du monomère comportant une chaîne grasse (monomère de formule (Ia), (Ib) ou (III)), et de préférence de 1,5 à 6 moles %.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en $C_1$-$C_4$.

Des polymères associatifs amphotères sont par exemple décrits et préparés dans la demande de brevet WO9844012.

Parmi les polymères associatifs amphotères selon l'invention, on préfère les terpolymères acide acrylique/ chlorure de (méth)acrylamidopropyl triméthyl ammonium/ méthacrylate de stéaryle.

Polymères associatifs de type non ionique

Selon l'invention, ils sont choisis de préférence parmi :

- **(1)** les celluloses modifiées par des groupements comportant au moins une chaîne grasse ;
  on peut citer à titre d'exemple :

  - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en $C_8$-$C_{22}$, comme le produit NATROSOL PLUS GRADE 330 CS (alkyles en $C_{16}$) vendu par la société AQUALON, ou le produit BERMOCOLL EHM 100 vendu par la société BEROL NOBEL,
  - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.

- **(2)** les hydroxypropylguars modifiés par des groupements comportant au moins une chaîne grasse tel que le produit ESAFLOR HM 22 (chaîne alkyle en $C_{22}$) vendu par la société LAMBERTI, les produits RE210-18 (chaîne alkyle en $C_{14}$) et RE205-1 (chaîne alkyle en $C_{20}$) vendus par la société RHONE POULENC.

- **(3)** les copolymères de vinyl pyrrolidone et de monomères hydrophobes à chaîne grasse dont on peut citer à titre d'exemple :

  - les produits ANTARON V216 ou GANEX V216 (copolymère vinylpyrrolidone /hexadécène) vendu par la société I.S.P.
  - les produits ANTARON V220 ou GANEX V220 (copolymère vinylpyrrolidone /eicosène) vendu par la société I.S.P.

- **(4)** les copolymères de méthacrylates ou d'acrylates d'alkyles en $C_1$-$C_6$ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.

- **(5)** les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.

- **(6)** les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.

- **(7)** les polymères à squelette aminoplaste éther possédant au moins une chaîne grasse, tels que les composés PURE THIX proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être

des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205 à fonction urée vendu par la société RHEOX ou encore les Rhéolates 208 , 204 ou 212, ainsi que l'Acrysol RM 184. On peut également citer le produit ELFACOS T210 à chaîne alkyle en $C_{12-14}$ et le produit ELFACOS T212 à chaîne alkyle en $C_{18}$ de chez AKZO.
Le produit DW 1206B de chez ROHM & HAAS à chaîne alkyle en $C_{20}$ et à liaison uréthanne, proposé à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.

Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate.
De tels polyéther polyuréthanes sont vendus notamment par la société ROHM & HAAS sous les appellations Aculyn 46 et Aculyn 44 [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

Les polymères associatifs de type non ionique, anionique, cationique ou amphotère sont utilisés de préférence en une quantité pouvant varier d'environ 0,1 à 10% en poids du poids total de la composition colorante. Plus préférentiellement, cette quantité varie d'environ 0,5 à 5% en poids, et encore plus particulièrement d'environ 1 à 3% en poids.

Polymères cationiques.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire.
Ce sont des produits connus. Ils sont notamment décrits dans les brevets français n° 2 505 348 ou 2 542 997. Parmi lesdits polymères, on peut citer :

**(1)** Les homopolymères ou copolymères dérivés d'esters ou d'amides acryliques ou méthacryliques et comportant au moins un des motifs de formules (I), (I1), (III) ou (IV) suivantes:

(I)

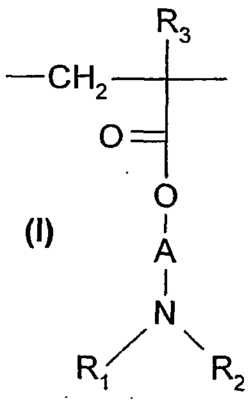

(II)

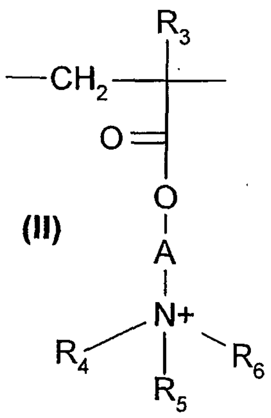

(III)

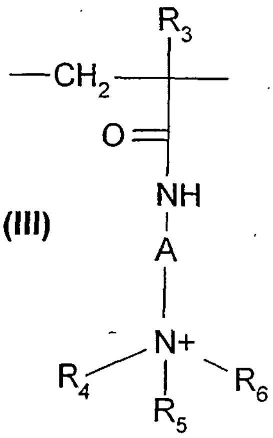

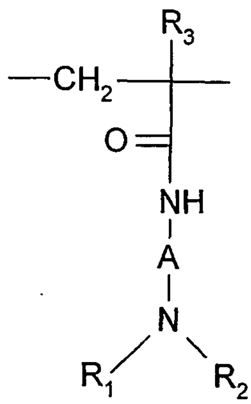

(IV)

dans lesquelles:

$R_3$ , identiques ou différents, désignent un atome d'hydrogène ou un radical $CH_3$;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone;
$R_1$ et $R_2$ , identiques ou différents, représentent hydrogène ou un groupe alkyle ayant de 1 à 6 atomes de carbone et de préférence méthyle ou éthyle;
X désigne un anion dérivé d'un acide minéral ou organique tel qu'un anion méthosulfate ou un halogénure tel que chlorure ou bromure.

Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs ($C_1$-$C_4$), des acides acryliques ou méthacryliques ou leurs esters, des vinyllactames tels que la vinylpyrrolidone ou le vinylcaprolactame, des esters vinyliques.
Ainsi, parmi ces polymères de la famille (1), on peut citer :

- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,

- les copolymères d'acrylamide et de chlorure de méthacryloyloxyéthyltriméthyl-ammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthyl-ammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylaminopropyle quaternisés tel que le produit vendu sous la dénomination "GAFQUAT HS 100" par la société ISP.

**(2)** Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.

**(3)** Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.

**(4)** Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

**(5)** Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

**(6)** Les cyclopolymères d'alkyl diallyl amine ou de dialkyl diallyl ammonium tels que les homopolymères ou copolymères comportant comme constituant principal de la chaîne des motifs répondant aux formules (V) ou (VI) :

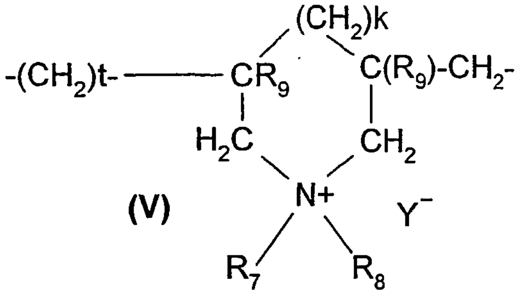

(V)

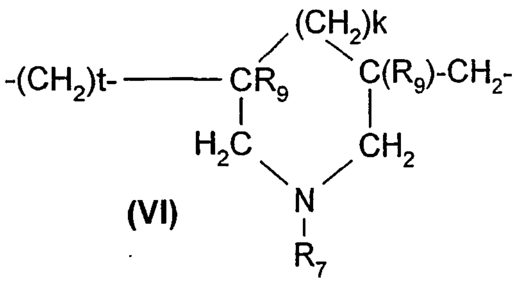

(VI)

formules dans lesquelles k et t sont égaux à 0 ou 1, la somme k + t étant égale à 1 ; $R_9$ désigne un atome d'hydrogène ou un radical méthyle ; $R_7$ et $R_8$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ($C_1$-$C_4$), ou $R_7$ et $R_8$ peuvent désigner conjointement avec l'atome d'azote auquel ils sont rattachés, des groupement hétérocycliques, tels que pipéridinyle ou morpholinyle ; $R_7$ et $R_8$ indépendamment l'un de l'autre désignent de préférence un groupement alkyle ayant de 1 à 4 atomes de carbone ; $Y^-$ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406. Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".

**(7)** Le polymère de diammonium quaternaire contenant des motifs récurrents répondant à la formule :

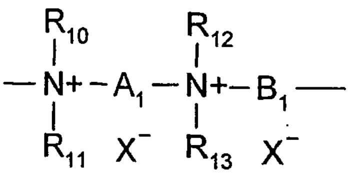

**(VII)**

formule (VII) dans laquelle :

$R_{10}$, $R_{11}$, $R_{12}$ et $R_{13}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{10}$, $R_{11}$, $R_{12}$ et $R_{13}$, ensemble ou séparément, constituent avec les atomes d'azote auxquels ils sont rattachés des hétérocycles contenant éventuellement un second hétéroatome autre que l'azote ou bien $R_{10}$, $R_{11}$, $R_{12}$ et $R_{13}$ représentent un radical alkyle en $C_1$-$C_6$ linéaire ou ramifié substitué par un groupement nitrile, ester, acyle, amide ou -CO-O- $R_{14}$-D ou -CO-NH-$R_{14}$-D où $R_{14}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et $X^-$ désigne un anion dérivé d'un acide minéral ou organique;
A1, $R_{10}$ et $R_{12}$ peuvent former avec les deux atomes d'azote auxquels ils sont rattachés un cycle pipérazinique ;

en outre si A1 désigne un radical alkylène ou hydroxyalkylène linéaire ou ramifié, saturé ou insaturé, B1 peut également désigner un groupement - $(CH_2)n-CO-D-OC-(CH_2)n-$ dans lequel n est compris entre 1 et 100 et de préférence entre 1 et 50, et D désigne :

a) un reste de glycol de formule : -O-Z-O-, où Z désigne un radical hydrocarboné linéaire ou ramifié ou un groupement répondant à l'une des formules suivantes :

$$-(CH_2-CH_2-O)x-CH_2-CH_2-$$

$$-[CH_2-CH(CH_3)-O]y-CH_2-CH(CH_3)-$$

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

$$-CH_2-CH_2-S-S-CH_2-CH_2- ;$$

d) un groupement uréylène de formule : -NH-CO-NH-.

De préférence, $X^-$ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule (VIII) suivante:

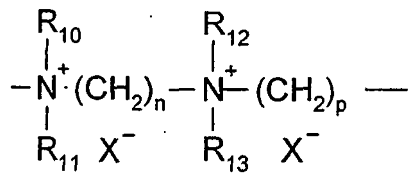

**(VIII)**

dans laquelle $R_{10}$, $R_{11}$, $R_{12}$ et $R_{13}$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

**(8)** Les polymères de polyammonium quaternaire constitués de motifs de formule (IX)

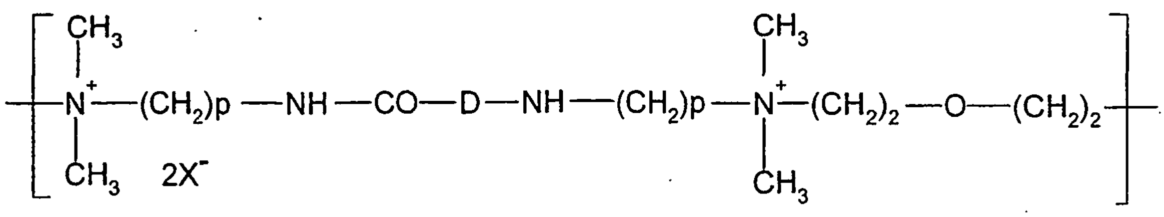

**(IX)**

dans laquelle :

p désigne un nombre entier variant de 1 à 6 environ,
D peut être nul ou peut représenter un groupement $-(CH_2)_r-CO-$ dans lequel r désigne un nombre égal à 4 ou à 7, et
$X^-$ est un anion dérivé d'un acide minéral ou organique.

Les polymères cationiques comportant des motifs de formule (IX) sont notamment décrits dans la demande de brevet EP-A-122 324 et peuvent être préparés selon les procédés décrits dans les brevets U.S.A. n° 4 157 388, 4 390 689; 4 702 906, 4 719 282.
Parmi ces polymères, on préfère ceux de masse moléculaire mesurée par RMN du Carbone 13 inférieure à 100000, et dans la formule de laquelle :

p est égal à 3, et,

a) D représente un groupement $-(CH_2)_4-CO-$, X désigne un atome de chlore, la masse moléculaire mesurée par RMN du Carbone 13 ( $RMN^{13}C$) étant d'environ 5600 ; un polymère de ce type est proposé par la société MIRANOL sous le nom de MIRAPOL-AD1,
b) D représente un groupement $-(CH_2)_7-CO-$, X désigne un atome de chlore, la masse moléculaire mesurée par RMN du Carbone 13 ( $RMN^{13}C$ ) étant d'environ 8100 ; un polymère de ce type est proposé par la société MIRANOL sous le nom de MIRAPOL-AZ1,
c) D désigne la valeur zéro, X désigne un atome de chlore, la masse moléculaire mesurée par RMN du Carbone 13, ( $RMN^{13}C$ étant d'environ 25500 ; un polymère de ce type est vendu par la société MIRANOL sous le nom MIRAPOL-A15,
d) un " Block Copolymer " formé de motifs correspondant aux polymères décrits aux alinéas a) et c), proposé par la société MIRANOL sous les noms MIRAPOL-9, (masse moléculaire $RMN^{13}C$, environ 7800) MIRAPOL-175, (masse moléculaire $RMN^{13}C$, environ 8000) MIRAPOL-95, (masse moléculaire $RMN^{13}C$, environ 12500).

Plus particulièrement encore, on préfère selon l'invention le polymère à motifs de formule (IX) dans laquelle p est égal à 3, D désigne la valeur zéro, X désigne un atome de chlore, la masse moléculaire mesurée par RMN du Carbone 13, ( $RMN^{13}C$ ) étant d'environ 25500.

**(9)** Les polymères quaternaires de vinylpyrrolidone et de vinylimidazole tels que par exemple les produits commercialisés sous les dénominations Luviquat FC 905, FC 550 et FC 370 par la société B.A.S.F.

**(10)** Les polyamines comme le Polyquart H vendu par HENKEL, référencé sous le nom de " POLYETHYLENEGLYCOL (15) TALLOW POLYAMINE " dans le dictionnaire CTFA.

**(11)** Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.
Parmi tous les polymères cationiques susceptibles d'être utilisés dans le cadre de la présente invention, on préfère mettre en oeuvre les polymères des familles (1), (6), (7) (8) et (11) et encore plus préférentiellement les polymères aux motifs récurrents de formules (W) et (U) suivantes :

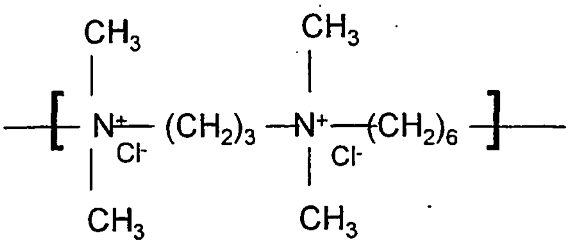

(W)

et notamment ceux dont le poids moléculaire, déterminé par chromatographie par perméation de gel, est compris entre 9500 et 9900;

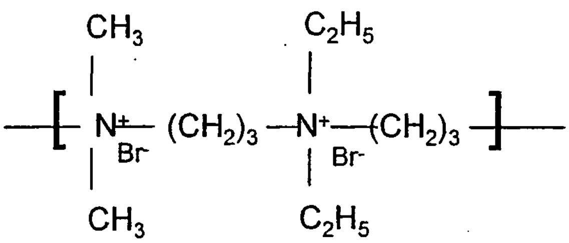

(U)

et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène $\alpha,\beta$-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :

(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
Le composé vinylique substitué contenant au moins un atome basique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.

(2) Les polymères comportant des motifs dérivant :

a) d'au moins un monomère choisi parmi les acrylamides ou les méthacrylamides substitués sur l'azote par un radical alkyle,

b) d'au moins un comonomère acide contenant un ou plusieurs groupements carboxyliques réactifs, et

c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.

Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.

Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.

Les comonomères basiques préférés sont des méthacrylates d'aminoéthyle, de butyl aminoéthyle, de N,N'-diméthylaminoéthyle, de N-tertio-butylaminoéthyle.

On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.

(3) Les polyaminoamides réticulés et alcoylés partiellement ou totalement dérivant de polyaminoamides de formule générale :

$$\{CO-R_{19}-CO-Z\} \quad \textbf{(X)}$$

dans laquelle $R_{19}$ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :

a) dans les proportions de 60 à 100 moles %, le radical

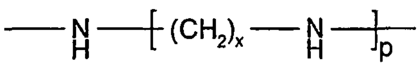

**(XI)**

où x=2 et p=2 ou 3, ou bien x=3 et p=2

ce radical dérivant de la diéthylène triamine, de la triéthylène tétraamine ou de la dipropylène triamine;

b) dans les proportions de 0 à 40 moles % le radical (XI) ci-dessus, dans lequel x=2 et p=1 et qui dérive de l'éthylènediamine, ou le radical dérivant de la pipérazine :

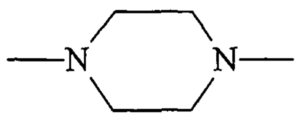

c) dans les proportions de 0 à 20 moles % le radical $-NH-(CH_2)_6-NH-$ dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.

Les acides carboxyliques saturés sont choisis de préférence parmi les acides ayant 6 à 10 atomes de carbone tels

que l'acide adipique, triméthyl-2,2,4-adipique et triméthyl-2,4,4-adipique, téréphtalique, les acides à double liaison éthylénique comme par exemple les acides acrylique, méthacrylique, itaconique.
Les alcanes sultones utilisées dans l'alcoylation sont .de préférence la propane ou la butane sultone, les sels des agents d'alcoylation sont de préférence les sels de sodium ou de potassium.

(4) Les polymères comportant des motifs zwittérioniques de formule :

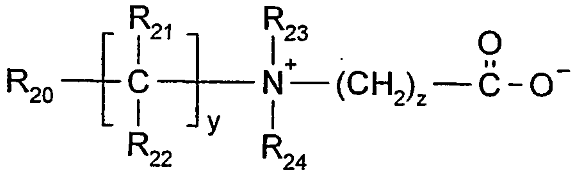

**(XII)**

dans laquelle $R_{20}$ désigne un groupement insaturé polymérisable tel qu'un groupement acrylate, méthacrylate, acrylamide ou méthacrylamide, y et z représentent un nombre entier de 1 à 3, $R_{21}$ et $R_{22}$ représentent un atome d'hydrogène, méthyle, éthyle ou propyle, $R_{23}$ et $R_{24}$ représentent un atome d'hydrogène ou un radical alkyle de telle façon que la somme des atomes de carbone dans $R_{23}$ et $R_{24}$ ne dépasse pas 10.
Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.

(5) les polymères dérivés du chitosane décrits notamment dans les brevets français N°-2137684 ou US-3879376, comportant des motifs monomères répondant aux formules (XIII), (XIV), (XV) suivantes réunies dans leur chaîne:

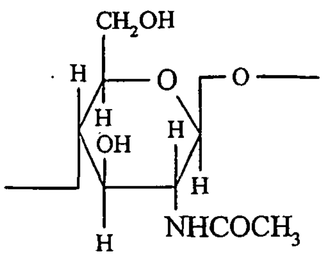

**(XIII)**

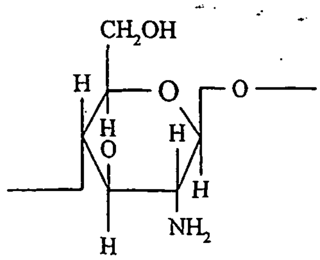

**(XIV)**

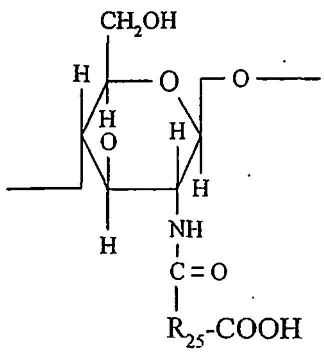

**(XV)**

le motif (XIII) étant présent dans des proportions comprises entre 0 et 30%, le motif (XIV) dans des proportions comprises entre 5 et 50% et le motif (XV) dans des proportions comprises entre 30 et 90%, étant entendu que dans ce motif (XV), $R_{25}$ représente un radical de formule:

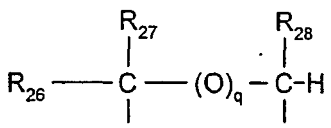

dans laquelle q désigne zéro ou 1 ;

si q=0, $R_{26}$, $R_{27}$ et $R_{28}$, identiques ou différents, représentent chacun un atome d'hydrogène, un reste méthyle,

hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux $R_{26}$, $R_{27}$ et $R_{28}$ étant dans ce cas un atome d'hydrogène ;
ou si q=1, $R_{26}$, $R_{27}$ et $R_{28}$ représentent chacun un atome d'hydrogène, ainsi que les sels formés par ces composés avec des bases ou des acides.

Des polymères de ce type plus particulièrement préférés comportent de 0 à 20% en poids de motifs (XIII), de 40 à 50% en poids de motifs (XIV), et de 40 à 50% en poids de motifs (XV) dans lequel $R_{25}$ désigne le radical $-CH_2-CH_2-$ ;

(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.

(7) Les polymères répondant à la formule générale (XI) tels que ceux décrits par exemple dans le brevet français 1 400 366 :

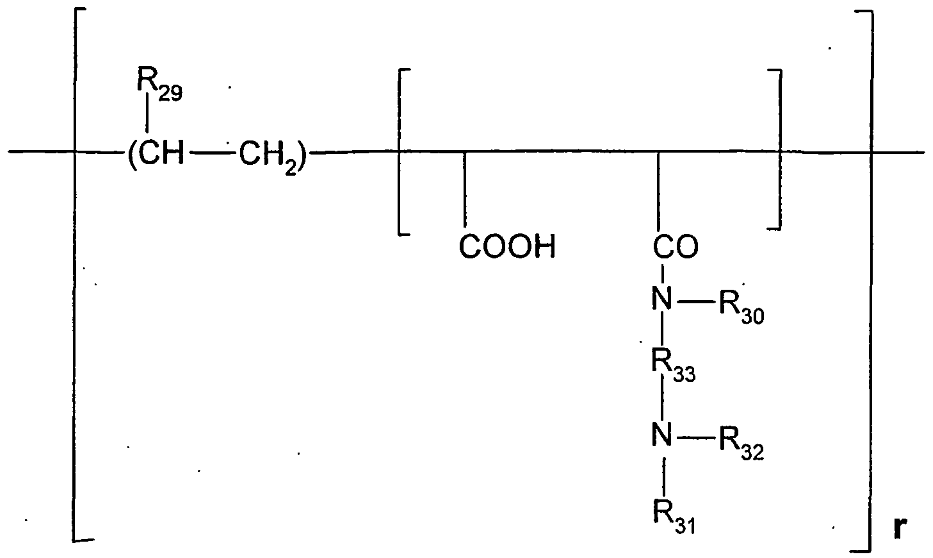

**(XVI)**

dans laquelle $R_{29}$ représente un atome d'hydrogène, un radical $CH_3O$, $CH_3CH_2O$, phényle, $R_{30}$ désigne l'hydrogène ou un radical alkyle inférieur tel que méthyle, éthyle, $R_{31}$ désigne l'hydrogène ou un radical alkyle inférieur tel que méthyle, éthyle, $R_{32}$ désigne un radical alkyle inférieur tel que méthyle, éthyle ou un radical répondant à la formule : $-R_{33}-N(R_{31})_2$, $R_{33}$ représentant un groupement $-CH_2-CH_2-$ , $-CH_2-CH_2-CH_2-$, $-CH_2-CH(CH_3)-$ , $R_{31}$ ayant les significations mentionnées ci-dessus,
ainsi que les homologues supérieurs de ces radicaux et contenant jusqu'à 6 atomes de carbone,
r est tel que le poids moléculaire est compris entre 500 et 6000000 et de préférence entre 1000 et 1000000.

(8) Des polymères amphotères du type -D-X-D-X- choisis parmi:

a) les polymères obtenus par action de l'acide chloracétique ou le chloracétate de sodium sur les composés comportant au moins un motif de formule :

-D-X-D-X-D- **(XVII)**

où D désigne un radical

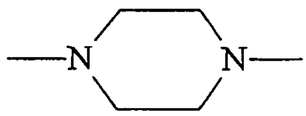

et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;

b) les polymères de formule :

-D-X-D-X- **(XVIII)**

où D désigne un radical

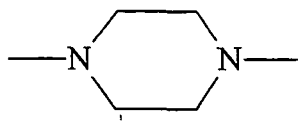

et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.

(9) Les copolymères alkyl($C_1$-$C_5$)vinyléther / anhydride maléique modifié partiellement par semiamidification avec une N,N-dialkylaminoalkylamine telle que la N,N-diméthylaminopropylamine ou par semiestérification avec une N, N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

Les compositions de l'invention comprennent de préférence un ou plusieurs tensioactifs qui peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

(i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkyl($C_6$-$C_{24}$) sulfosuccinates, les alkyl($C_6$-$C_{24}$) éthersulfosuccinates, les alkyl($C_6$-$C_{24}$) amidesulfosuccinates ; les alkyl($C_6$-$C_{24}$) sulfoacétates ; les acyl($C_6$-$C_{24}$) sarcosinates et les acyl($C_6$-$C_{24}$) glutamates. On peut également utiliser les esters d'alkyl ($C_6$-$C_{24}$)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl

désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène, et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensio-actifs non-ioniques rentrant particulièrement bien dans le cadre de la présente invention.

(iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) betaïnes ou les alkyl ($C_8$-$C_{20}$) amidoalkyl ($C_1$-$C_6$) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

$$R_2\ \text{-CONHCH}_2\text{CH}_2\ \text{-N}(R_3)(R_4)(\text{CH}_2\text{COO}^-)$$

dans laquelle : $R_2$ désigne un radical alkyle d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ; et

$$R_2'\text{-CONHCH}_2\text{CH}_2\text{-N(B)(C)}$$

dans laquelle :

B représente -$CH_2CH_2OX'$, C représente -$(CH_2)_z$ -Y', avec z = 1 ou 2,
X' désigne le groupement -$CH_2CH_2$-COOH ou un atome d'hydrogène
Y' désigne -COOH ou le radical -$CH_2$ - CHOH - $SO_3H$
$R_2'$ désigne un radical alkyle d'un acide $R_9$ -COOH présent dans l'huile de coprah ou dans l'huile de lin hydrolysée, un radical alkyle, notamment en $C_7$, Cg, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL®

C2M concentré par la société RHODIA CHIMIE.

(iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs présents dans la composition selon l'invention peuvent varier de 0,01 à 40% et de préférence de 0,5 à 30% du poids total de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Dans la composition prête à l'emploi avec agent oxydant, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.

On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition [composition sans oxydant ou avec oxydant], est généralement compris entre les valeurs 2 et 12. Il est de préférence compris entre 3 et 11, et plus particulièrement compris entre 7 et 10. IL peut être ajusté à la valeur désirée au moyen d'agents acidifiants ou alcalinisants ou de tampons bien connus de l'état de la technique en teinture des fibres kératiniques.

Plus préférentiellement lorsque la composition contient un agent oxydant pour l'éclaircissement des fibres, le pH du mélange prêt à l'emploi est supérieur à 7 et encore plus préférentiellement supérieur à 8.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les éthylènediamines oxyéthylénées et/ou oxypropylénées, les hydroxydes de sodium ou de potassium et les composés de formule (XIX) suivante :

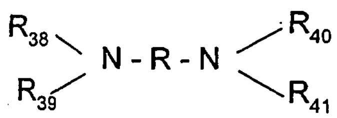

**(XIX)**

dans laquelle R est un reste propylène éventuellement substitué par un groupement hydroxyle ou un radical alkyle en $C_1$-$C_4$ ; $R_{38}$, $R_{39}$, $R_{40}$ et $R_{41}$, identiques ou différents, représentent un atome d'hydrogène, un radical alkyle en $C_1$-$C_4$ ou hydroxyalkyle en $C_1$-$C_4$.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Parmi les tampons, on peut citer ceux vendus par la société MERCK sous la marque TITRISOL, tels que que le tampon phosphate ($KH_2PO_4$ à 0,026 mole/l, $Na_2HPO_4$ à 0,041 mole/l), et le tampon borate ($H_3BO_3$ à 0,05 mole/l, KCl à 0,05 mole/l, NaOH à 0,022 mole/l).

Le procédé de teinture selon l'invention est conduit à température ambiante, et consiste à appliquer la composition de teinture selon l'invention, sans oxydant, ou avec oxydant (réalisée extemporanément au moment de l'emploi), sur les fibres kératiniques sèches ou humides, et à la laisser agir pendant un temps de pause variant de 5 secondes à 60 minutes environ, plus préférentiellement de 10 secondes à 5 minutes environ, et plus particulièrement de 30 secondes à 2 minutes environ, à rincer les fibres, puis éventuellement à les laver au shampooing, puis à les rincer à nouveau, et à les sécher.

Le procédé selon l'invention présente l'avantage de ne nécessiter que des temps d'application très courts, de l'ordre de 10 secondes à 5 minutes et plus particulièrement de 30 secondes à 2 minutes.

Il est possible de conduire le procédé à des températures plus élevées telles que celles produites par un casque

de coiffure, 40°C environ, ou par un brushing, 70-80°C environ.

Des exemples concrets illustrant l'invention sont indiqués ci-après, sans pour autant présenter un caractère limitatif.

EXEMPLES 1 à 4

On a préparé les compositions de teinture directe suivantes :
]

[teneurs exprimées en grammes Matière Active (MA)

| EXEMPLE N° | COLORANT DIRECT DE FORMULE (I) 0,5g | SUPPORT] | TAMPON P* ou B * | EAU q.s.p. |
|---|---|---|---|---|
| 1 | (I)1 | ] | P | 100 |
| 2 | (I)1 | ] | B | 100 |
| 3 | (I)8 | ] | P | 100 |
| 4 | (I)8 | ] | B | 100 |

SUPPORT] :

| | |
|---|---|
| Hydroxyéthylcellulose | 0,384 g |
| Tensio actif non ionique : | |
| Alkyl(C8/C10 50/50) polyglucoside | |
| en solution aqueuse à 60% | 3 g MA |
| Alcool benzylique | 4 g |
| Polyéthylène glycol (8OE) | 6 g |
| Conservateur : | |
| p-hydroxybenzoate de méthyle, | |
| butyle, éthyle, propyle et isobutyle | 0,032 g |
| Tampon 7 phosphate | 50 g |
| ou Tampon 9 borate | 50 g |
| Tampon P* = tampon 7 phosphate | |
| Tampon B* = tampon 9 borate | |

Chacune des compositions des exemples 1 à 4 a été appliquée, d'une part sur des mèches de cheveux gris naturels à 90% de blancs, d'autre part sur des mèches de cheveux gris permanentés à 90% de blancs, pendant 20 minutes à température ambiante (20°C).
A l'issue du temps de pause, les mèches de cheveux ont été rincées, puis séchées.
La couleur a ensuite été mesurée au colorimètre MINOLTA CM2002 dans le système L* a* b* .

Dans le système L* a* b* , les 3 paramètres désignent respectivement l'intensité (L* ), la nuance (a* ), et la saturation (b* ).

Selon ce système, plus la valeur de L est élevée, plus la couleur est claire ou peu intense. Inversement, plus la valeur de L est faible, plus la couleur est foncée ou très intense.
a* et b* indiquent deux axes de couleurs, a* indique l'axe de couleur vert/rouge et b* l'axe de couleur bleu/jaune. Des valeurs proches de zéro pour a* et b* correspondent à des nuances grises.
La sélectivité de la coloration ΔE peut être calculée en appliquant l'équation suivante :

$$\Delta E = \sqrt{(L^* - L_o^*)^2 + (a^* - a_o^*)^2 + (b^* - b_o^*)^2}$$

Dans cette équation, ΔE représente la différence de couleur entre deux mèches, (dans le cas présent la sélectivité de la coloration), L* , a* , et b* représentent respectivement l'intensité, la nuance et la saturation de la mèche de cheveux naturels teinte, $L_O$* , $a_O$* et $b_O$* représentant respectivement l'intensité, la nuance et la saturation de la mèche de cheveux permanentés teinte.
Plus la valeur de ΔE est importante, plus la différence de couleur entre les deux mèches est importante, et plus la sélectivité de la teinture est importante.

Les résultats ont été réunis dans le tableau (I) ci-dessous.

Tableau (I)

| EXEMPLES | Types de Cheveux | L* | a* | b* | **Sélectivité** (△E entre cheveux naturels et permanentés) |
|---|---|---|---|---|---|
| 1 | naturels | 29.77 | 7.56 | -8.28 | |
| 1 | permanentés | 27.64 | 9.26 | -11.01 | **3.86** |
| 2 | naturels | 31.68 | 9.24 | -11.35 | |
| 2 | permanentés | 27.56 | 9.54 | -12.65 | **4.33** |
| 3 | naturels | 49.42 | 19.10 | 37.27 | |
| 3 | permanentés | 45.76 | 20.31 | 38.05 | **3.93** |

Conclusion : ces résultats démontrent clairement que les teintures selon l'invention sont puissantes et peu sélectives.

EXEMPLES 5 à 6

On a préparé les compositions de teinture directe suivantes :

[teneurs exprimées en grammes Matière Active (MA)]

| EXEMPLE N° | COLORANT DIRECT DE FORMULE (I) 0,5 g | TAMPON BORATE pH 9 q.s.p. |
|---|---|---|
| 5 | (I)1 | 100 |
| 6 | (I)8 | 100 |

Chacune des compositions des exemples 5 et 6 a été appliquée, d'une part sur des mèches de cheveux gris naturels à 90% de blancs, d'autre part sur des mèches de cheveux gris permanentés à 90% de blancs, pendant 30 secondes à température ambiante (20°C).
A l'issue du temps de pause, les mèches de cheveux ont été rincées, puis séchées.
La sélectivité de la coloration a été évaluée suivant la même technique que précédemment dans les exemples 1 à 4.
Les résultats sont réunis dans le tableau (II) ci-dessous :

Tableau (II)

| EXEMPLES | Types de Cheveux | L* | a* | b* | **Sélectivité** (△E entre cheveux naturels et permanentés) |
|---|---|---|---|---|---|
| 5 | naturels | 38.10 | 7.63 | -9.28 | |
| 5 | permanentés | 32.56 | 7.72 | -8.41 | **5.61** |
| 6 | naturels | 51.77 | 9.17 | 25.99 | |
| 6 | permanentés | 54.76 | 11.07 | 31.30 | **6.38** |

Conclusion : ces résultats démontrent clairement que les teintures selon l'invention sont puissantes et peu sélectives.

EXEMPLES 7 à 8

On a préparé les compositions de teinture directe éclaircissantes suivantes :

[teneurs exprimées en grammes Matière Active (MA)]

| EXEMPLES | 7 | 8 |
|---|---|---|
| Hydroxyéthylcellulose | 1,39 | 1,39 |
| Ammoniaque (40% $NH_4OH$) | 2,08 | 2,08 |
| Eau oxygénée 40 volumes | 60 | 60 |
| Colorant direct de formule (I)1 | 0,52 | |
| Colorant direct de formule (I)8 | | 0,52 |
| Eau déminéralisée q.s.p. | 100 | 100 |

Chacune des compositions des exemples 7 et 8 a été appliquée, d'une part sur des mèches de cheveux gris naturels à 90% de blancs, d'autre part sur des mèches de cheveux gris permanentés à 90% de blancs, pendant 35 minutes à température ambiante (20°C).
A l'issue du temps de pause, les mèches de cheveux ont été rincées, puis séchées.
La sélectivité de la coloration a été évaluée suivant la même technique que précédemment dans les exemples 1 à 4.
Les résultats sont réunis dans le tableau (III) ci-dessous :

Tableau (III)

| EXEMPLES | Types de Cheveux | L* | a* | b* | **Sélectivité** (△E entre cheveux naturels et permanentés) |
|---|---|---|---|---|---|
| 7 | naturels | 33.99 | 10.20 | -10.99 | |
| 7 | permanentés | 32.06 | 11.10 | -11.29 | **2.15** |
| 8 | naturels | 48.64 | 22.78 | 36.59 | |
| 8 | permanentés | 48.66 | 27.94 | 41.88 | **7.39** |

Conclusion : ces résultats démontrent clairement que les teintures selon l'invention sont puissantes et peu sélectives.

EXEMPLES 9 à 14

On a préparé d'autres compositions de teinture directe éclaircissantes avec les colorants de formules (I)2, (I) 3, (I)4, (I)5, (I)6, et (I)7 décrites ci-dessus, identiques à celles décrites dans les exemples 7 et 8. Appliquées de la même façon que dans les exemples 7 et 8, les teintures obtenues ont été d'un rouge puissant.

## Revendications

1. Utilisation pour la teinture des fibres kératiniques humaines et plus particulièrement des cheveux, à titre de colorants directs, dans une composition cosmétique, de composés de formules (I), (II), ou (III) suivantes :

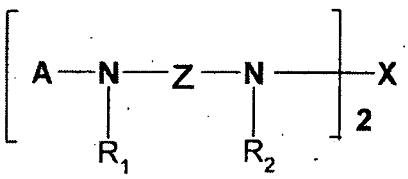

(I)

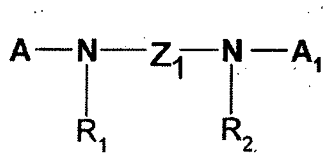

(II)

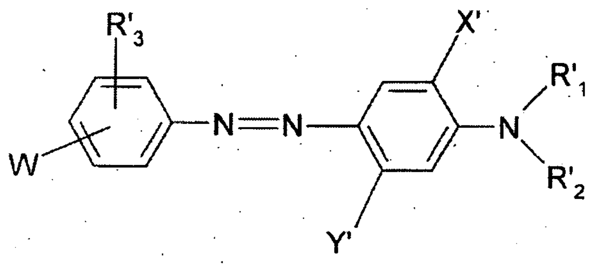

(III)

**formules (I) ou (II)** dans lesquelles :

- A et A1, indépendamment l'un de l'autre désignent un radical de formule (a) suivante

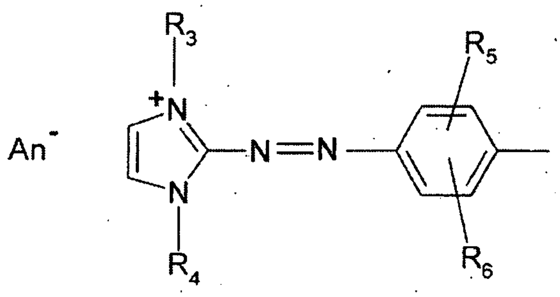

(a)

- Z désigne un radical aliphatique ou aromatique,
- $Z_1$ désigne un radical alkyle,
- $R_1$ et $R_2$, indépendamment l'un de l'autre, désignent un atome d'hydrogène, ou un radical ($C_1$-$C_4$)alkyl, ou ($C_1$-$C_4$)alkyl substitué par un ou plusieurs atomes d'halogène, un radical hydroxyl, carboxyl, cyano, un radical ($C_1$-$C_4$)alcoxy, un radical ($C_1$-$C_4$)alcoxy substitué par un ou plusieurs radicaux hydroxyl, ou ($C_1$-$C_4$)alcoxy, un radical amino, alkylamino, dialkylamino, aminocarbonyl, phényl, phénoxy ou phénylaminocarbonyl, dans lequel le radical phényl est non substitué ou substitué par un radical ($C_1$-$C_4$)alkyl, ($C_1$-$C_4$)alcoxy ou phénoxy, ou encore $R_1$ et $R_2$ forment ensemble, avec les deux atomes d'azote qui les portent et le radical Z, un cycle pipérazinique,
- X est un radical de pontage choisi parmi : -CO- ; $-CO-CH_2-CH_2-CO-$ ; -CO-CO- ; 1,4-dicarbonylphényl ; $-CH_2-CH_2-$ ; ou une triazine de formules (b) ou (c) suivantes:

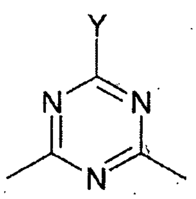

(b)

ou

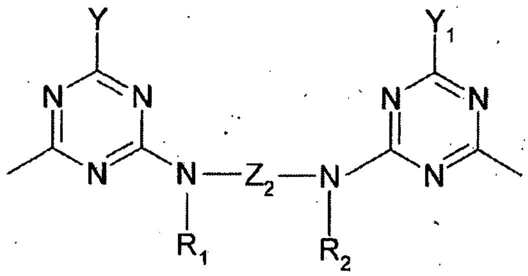

(c)

dans lesquelles :

Y et Y1, indépendamment l'un de l'autre désignent un atome d'halogène, ou un radical hydroxyl, ou amino, ou monoalkylamino, ou dialkylamino, ou 1-pipéridino, ou morpholino, ou 1-pipérazino, le radical pipérazino étant non substitué, ou substitué sur l'atome d'azote non attaché au cycle triazine par un radical ($C_1$-$C_4$) alkyl, lesdits radicaux alkyls étant non substitués ou substitués par hydroxyl, amino, mono-($C_1$-$C_4$)alkylamino ou di-($C_1$-$C_4$)alkylamino,

- $Z_2$ désigne un radical ($C_2$-$C_8$)alkylène ou forme, avec les deux atomes d'azote adjacents et les radicaux $R_1$ et $R_2$, un cycle pipérazinique,
- dans le radical de formule (a),
- $R_3$ et $R_4$, indépendamment l'un de l'autre, désignent un atome d'hydrogène, ou un radical ($C_1$-$C_4$)alkyl, ou ($C_1$-$C_4$)alkyl substitué par un ou plusieurs atomes d'halogène, un radical hydroxyl, carboxyl, cyano, un radical ($C_1$-$C_4$)alcoxy, un radical ($C_1$-$C_4$)alcoxy substitué par un radical hydroxyl ou ($C_1$-$C_4$)alcoxy, un radical amino, alkylamino, dialkylamino, aminocarbonyl, phényl, phénoxy ou phénylaminocarbonyl, dans lequel le radical phényl est non substitué ou substitué par un radical ($C_1$-$C_4$)alkyl, ($C_1$-$C_4$)alcoxy ou phénoxy,
- $R_5$ et $R_6$, indépendamment l'un de l'autre, désignent un atome d'hydrogène, un radical ($C_1$-$C_4$)alkyl ou ($C_1$-$C_4$) alcoxy éventuellement substitués par un radical hydroxyl, carboxyl, halogène, cyano, ($C_1$-$C_4$)alcoxy éventuellement substitué par un radical hydroxyl ou ($C_1$-$C_4$)alcoxy, un radical amino, alkylamino, dialkylamino, aminocarbonyl, phényl, phénoxy ou phénylaminocarbonyl, dans lequel le radical phényl est non substitué ou substitué par un radical ($C_1$-$C_4$)alkyl, ($C_1$-$C_4$)alcoxy ou phénoxy,
- $An^-$ désigne un anion.

**formule (III)** dans laquelle,

- le nombre de charges cationiques est de deux,
- X' et Y', indépendamment l'un de l'autre, désignent hydrogène, halogène, ($C_1$-$C_4$)alkyl, ($C_1$-$C_4$)alcoxy, ($C_1$-$C_4$) alkylcarbonylamino, arylcarbonylamino, uréido, ou aryluréido,
- $R'_1$ désigne hydrogène, un radical alkyl ou aryl substitués, un radical alkyl ou aryl non substitués, ou la même désignation que $R'_2$
- $R'_2$ est un radical de formule (d) suivante:

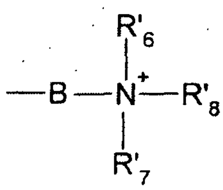

(d)

dans laquelle :
- B désigne un radical alkylène linéaire ou ramifié,
- $R'_6$ désigne hydrogène ou alkyl substitué ou non substitué,
- $R'_7$ et $R'_8$, indépendamment l'un de l'autre désignent alkyl substitué ou non substitué,
- $R'_6$ et $R'_7$, ensemble avec l'azote, forment un cycle à 5, 6, ou 7 chaînons, substitué ou non substitué, pouvant contenir d'autres hétéroatomes, ou bien $R'_6$ et $R'_7$ et $R'_8$ forment ensemble un cycle pyridinium,
- $R'_3$ désigne hydrogène, halogène, ($C_1$-$C_4$)alkyl, ($C_1$-$C_4$)alcoxy,

- W est un radical de formule (e) suivante :

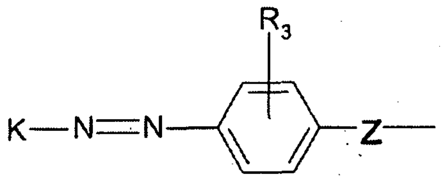

(e)

dans laquelle:

- K est un radical de couplage,
- Z désigne un radical de pontage choisi parmi les radicaux de formules :

$-NR'_9-CO-$;

$-CO-NR'_9-NR'_9-CO-$;

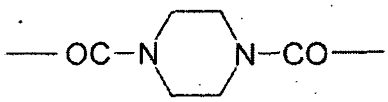

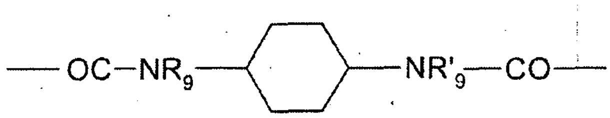

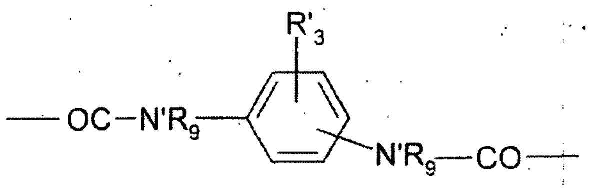

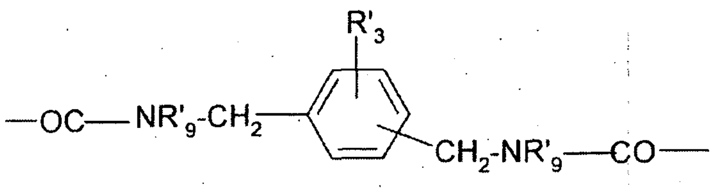

ou bien

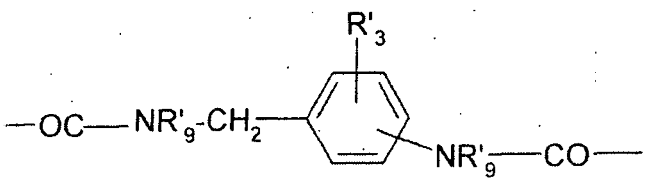

et dans lesquels R'$_9$ désigne, hydrogène, ($C_2$-$C_4$)alkylène non substitué ou substitué, le radical alkylène étant linéaire ou ramifié et pouvant être interrompu par un ou plusieurs groupements choisis parmi : -NR'9-, -O-, -S-.

2. Utilisation selon la revendication 1, **caractérisée par le fait que** dans la formule (I),

- $R_1$ et $R_2$, indépendamment l'un de l'autre, désignent hydrogène, ($C_1$-$C_4$)alkyl substitué par hydroxyl ou ($C_1$-$C_4$) alcoxy et plus particulièrement encore désignent hydrogène ou méthyle,
- Z désigne un radical alkyle en $C_2$-$C_8$ linéaire ou ramifié ou cyclique, éventuellement substitué par un hydroxy, alcoxy, halogène, la chaîne dudit radical étant éventuellement interrompue par un groupe -O- ou -$NR_1$- ; un radical 1,4-phényl, un radical 1,4-naphtyl éventuellement substitué par un alkyle, alcoxy, halogène ; Z pouvant formé avec $R_1$, $R_2$ et les 2 atomes d'azote, un cycle pipérazine, et préférentiellement, Z désigne un radical phényl non substitué, un radical phényl ou naphtyl substitué par 1 ou 2 radicaux méthyl ou méthoxy, un radical pipérazine par liaison avec $R_1$, $R_2$ et les 2 atomes d'azote, un radical ($C_2$-$C_4$)alkylène non subsitué ou substitué par un ou 2 hydroxyl,
- X désigne un groupement de formule (b).

3. Utilisation selon la revendication 1, **caractérisée par le fait que** dans la formule (II), -Z1 désigne un radical alkyle en $C_2$-$C_8$ linéaire ou ramifié ou cyclique, éventuellement substitué par un hydroxy, alcoxy, halogène, la chaîne dudit radical étant éventuellement interrompue par un groupe -O- ou -$NR_1$- ; un cycle pipérazine formé avec $R_1$, $R_2$ et les deux atomes d'azote, et préférentiellement, $Z_1$ désigne un radical ($C_2$-$C_6$)alkylène non substitué ou substitué par un ou plusieurs hydroxyl, un cycle pipérazine formé avec $R_1$, $R_2$ et les deux atomes d'azote ; et plus particulièrement encore un radical ($C_2$-$C_4$)alkylène non substitué,

- $R_3$ et $R_4$ désignent méthyl ou éthyl, et $R_5$ et $R_6$ désignent hydrogène, méthyl, ou méthoxy.

4. Utilisation selon la revendication 1, **caractérisée par le fait que** dans la formule (III), B désigne éthylène, n-propylène, isopropylène ou n-butylène,

K désigne un composé de couplage choisi parmi ceux de formule (f), (g) ou (h) suivantes

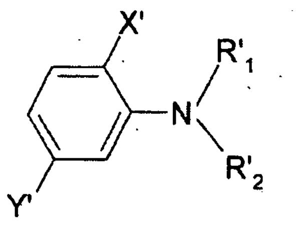

**(f)**

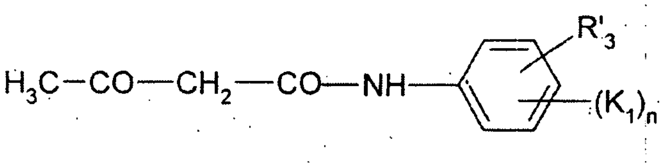

**(g)**

ou

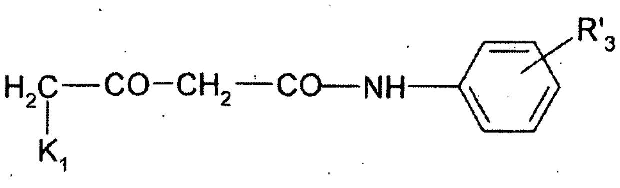

**(h)**

dans lesquelles,
-X', Y' et R'$_1$, R'$_2$ et R'$_3$, ont la même désignation que dans la formule (III),

-n est égal à 1 ou 2,
-$K_1$ désigne le radical de formule :

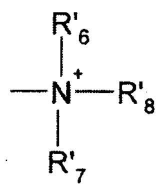

**5.** Utilisation selon l'une quelconque des revendications 1 ou 2, **caractérisée par le fait que** le composé de formule (I) ou de formule (II) est choisi parmi ceux de formules (I)1 à (I)7 suivantes :

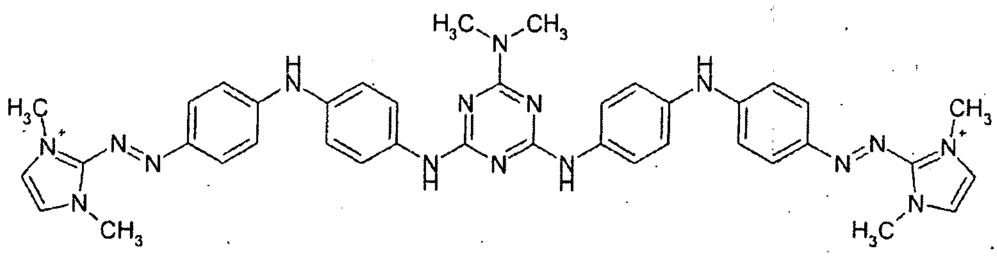

**(I)1**

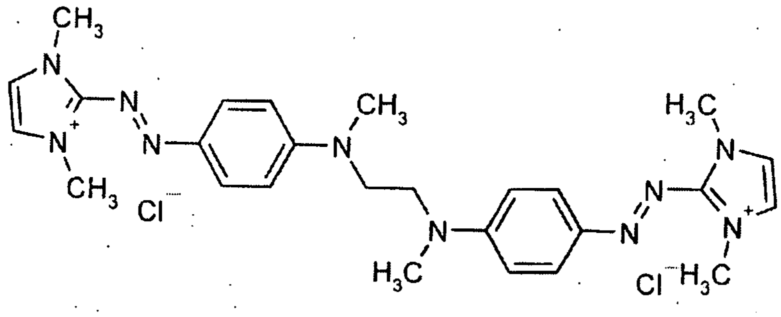

**(I)2**

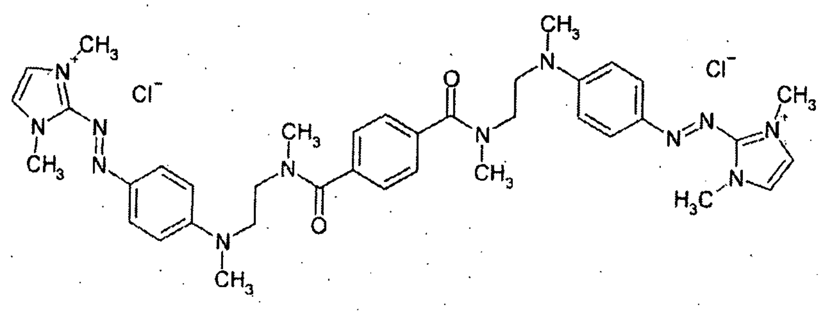

**(I)3**

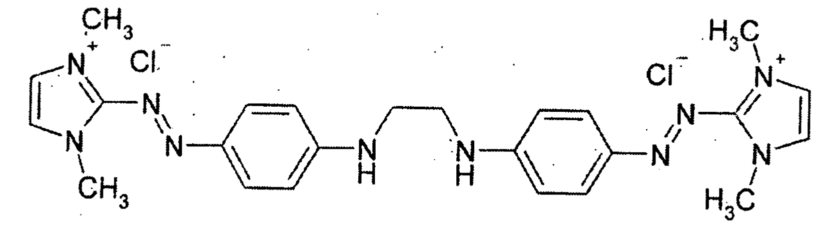

(I)4

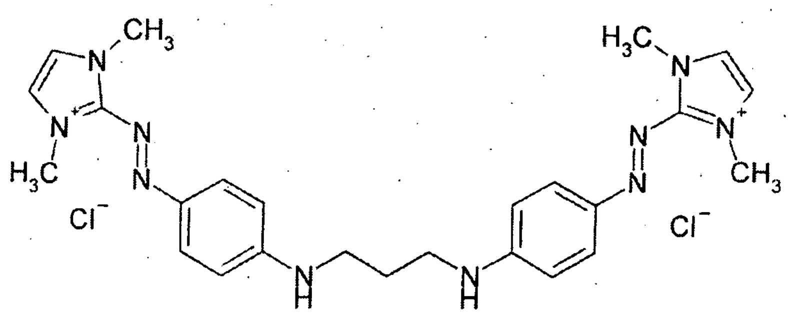

(I)5

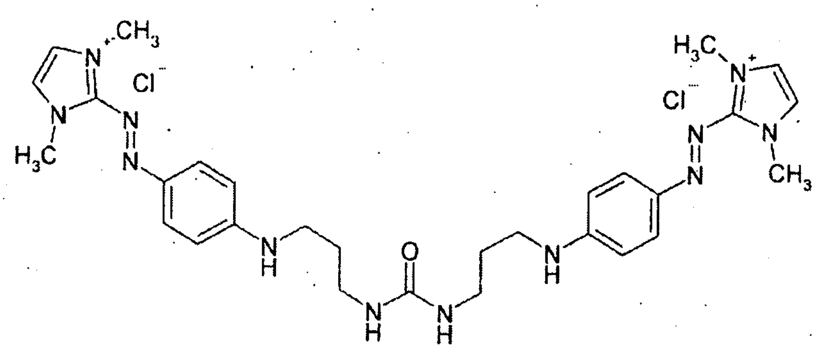

(I)6

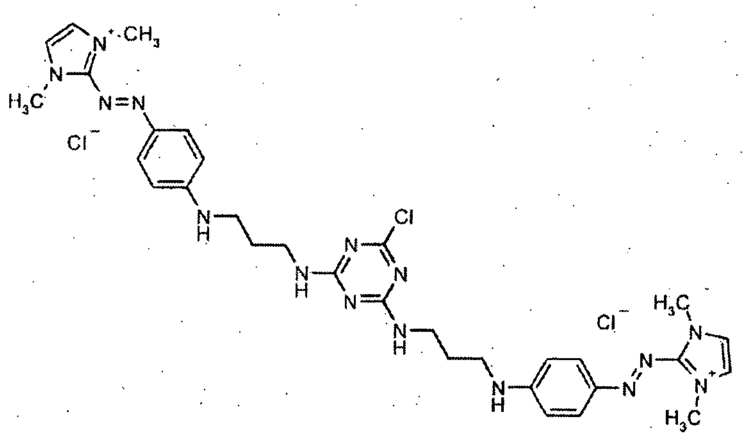

**(I)7**

**6.** Utilisation selon l'une quelconque des revendications 1 ou 4, **caractérisée par le fait que** le composé de formule (III) est le composé suivant :

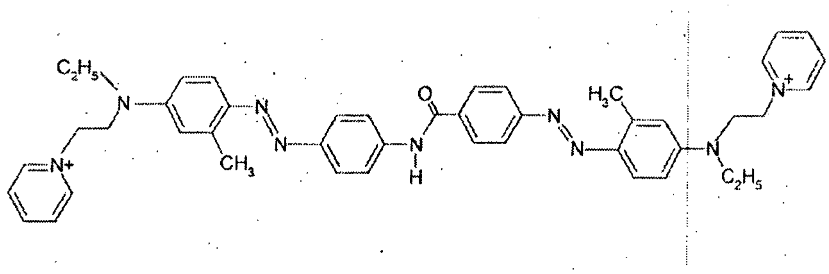

**7.** Composition de teinture pour fibres kératiniques humaines et plus particulièrement des cheveux, **caractérisée par le fait qu'**elle comprend une quantité efficace d'au moins un colorant direct de formules (I), (II), ou (III) telles que définies à l'une quelconque des revendications précédentes, un ou plusieurs tensioactifs indifféremment choisis, seuls ou en mélanges, parmi les tensioactifs anioniques, non ioniques, amphotères, zwittérioniques et cationiques.

**8.** Composition selon la revendication 7, **caractérisée par le fait que** le ou les colorants directs de formules (I), (II), ou (III) sont présents en une quantité variant de 0,01 à 40% en poids par rapport au poids total de la composition.

**9.** Composition selon la revendication 8, **caractérisée par le fait que** le ou les colorants directs de formules (I), (II), ou (III) sont présents en une quantité variant de 0,1 à 20% en poids par rapport au poids total de la composition.

**10.** Composition selon l'une quelconque des revendications 7 à 9, **caractérisée par le fait qu'**elle renferme en outre au moins un agent oxydant.

**11.** Composition selon la revendication 10, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

**12.** Composition selon la revendication 11, **caractérisée par le fait que** l'agent oxydant est le peroxyde d'hydrogène.

**13.** Composition selon la revendication 12, **caractérisée par le fait qu'**il s'agit d'une solution d'eau oxygénée dont le titre varie de 1 à 40 volumes.

**14.** Composition selon l'une quelconque des revendications 7 à 13, **caractérisée par le fait qu'**elle possède un pH compris entre 2 et 12, de préférence compris entre 3 et 11 et plus particulièrement compris entre 7 et 10.

**15.** Composition selon l'une quelconque des revendications 7 à 14, **caractérisée par le fait qu'**elle contient en outre

au moins, dans la proportion de 0,01 à 10% en poids par rapport au poids total de la composition, d'un polymère cationique ou amphotère choisi parmi :

- 1/ les homopolymères de chlorure de diméthyldiallylammonium;
- 2/ les polymères au motifs récurrents de formules (W) ou (U) suivantes :

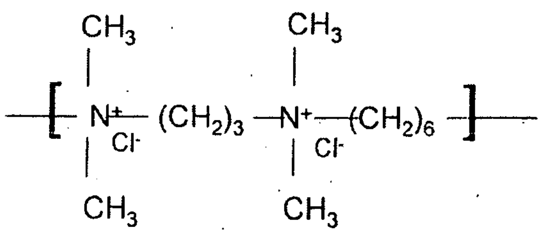

**(W)**

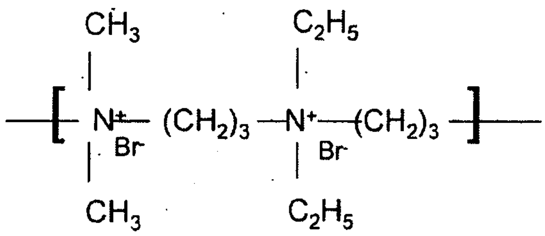

**(U)**

- 3/ les polymères aux motifs de formule (IX) suivante :

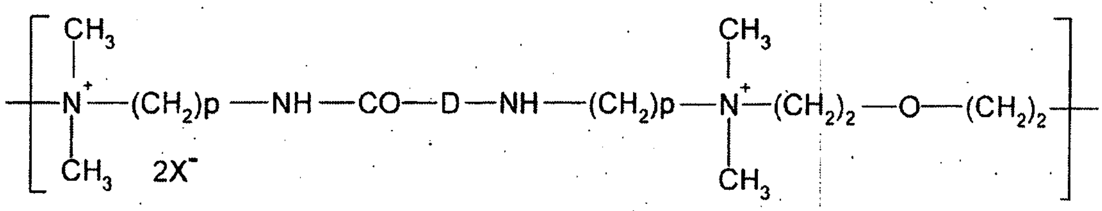

**(IX)**

pour laquelle, p est égal à 3, et,

a) D désigne la valeur zéro, X désigne un atome de chlore,
b) D représente un groupement $-(CH_2)_4-CO-$, X désigne un atome de chlore,
c) D représente un groupement $-(CH_2)_7-CO-$, X désigne un atome de chlore,
d) un " Block Copolymer " formé de motifs correspondant aux polymères décrits aux alinéas a) et b);

- 4/ les copolymères d'acide acrylique et de chlorure de diméthyldiallylammonium.

**16.** Procédé de teinture des fibres kératiniques et en particulier des cheveux, **caractérisé par le fait qu'**il consiste à appliquer la composition de teinture telle que décrite à l'une quelconque des revendications 1 à 6, sur les fibres sèches ou humides, et à la laisser agir pendant un temps de pause variant de 5 secondes à 60 minutes, à rincer les fibres, puis éventuellement à les laver au shampooing, puis à les rincer à nouveau, et à les sécher.

**17.** Procédé de teinture des fibres kératiniques et en particulier dés cheveux, **caractérisé par le fait qu'**il consiste à appliquer la composition de teinture telle que décrite à l'une quelconque des revendications 7 à 15 sur les fibres sèches ou humides, et à la laisser agir pendant un temps de pause variant de 5 secondes à 60 minutes, à rincer les fibres, puis éventuellement à les laver au shampooing, puis à les rincer à nouveau, et à les sécher.

**18.** Procédé selon l'une des revendications 16 ou 17, **caractérisé par le fait que** lorsque la composition comprend un

oxydant, on mélange l'oxydant à la composition juste avant l'application sur les fibres.

**19.** Procédé selon l'une quelconque des revendications 16 à 18, **caractérisé par le fait que** le temps de pause varie de 10 secondes à 5 minutes et plus particulièrement de 30 secondes à 2 minutes.

**20.** Dispositif pour la teinture directe éclaircissante des fibres kératiniques humaines et en particulier des cheveux, **caractérisé par le fait qu'**il comprend un premier compartiment renfermant, dans un milieu approprié pour la teinture, au moins un colorant direct de formules (I), (II), ou (III) définies à l'une quelconque des revendications 1 à 6 et un deuxième compartiment renfermant un agent oxydant.

## Claims

**1.** Use, for dyeing human keratin fibres, and more particularly the hair, as direct dyes, in a cosmetic composition, of compounds of formula (I), (II) or (III) below:

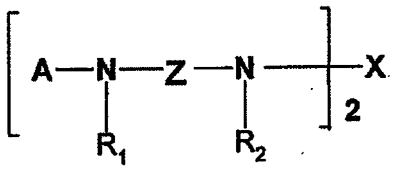

(I)

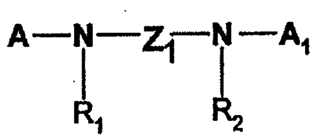

(II)

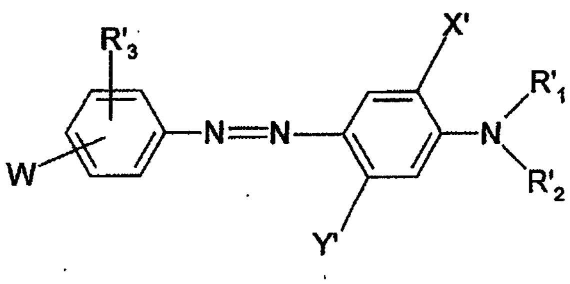

(III)

in which formula (I) or (II):

- A and A1, independently of each other, denote a radical of formula (a) below

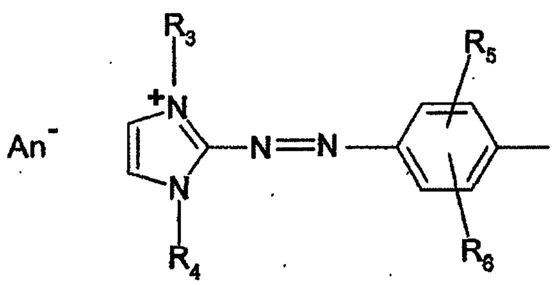

(a)

- Z denotes an aliphatic or aromatic radical,
- $Z_1$ denotes an alkyl radical,
- $R_1$ and $R_2$, independently of each other, denote a hydrogen atom or a ($C_1$-$C_4$)alkyl radical, or a ($C_1$-$C_4$)alkyl radical substituted with one or more halogen atoms, a hydroxyl, carboxyl or cyano radical, a ($C_1$-$C_4$)alkoxy radical, a ($C_1$-$C_4$)alkoxy radical substituted with one or more hydroxyl or ($C_1$-$C_4$)alkoxy radicals, or an amino, alkylamino, dialkylamino, aminocarbonyl, phenyl, phenoxy or phenylaminocarbonyl radical, in which the phenyl radical is unsubstituted or substituted with a ($C_1$-$C_4$) alkyl, ($C_1$-$C_4$) alkoxy or phenoxy radical,

or $R_1$ and $R_2$ form, together with the two nitrogen atoms that bear them and the radical Z, a piperazine ring,
- X is a bridging radical chosen from: -CO-; -CO-$CH_2$-$CH_2$-CO-; -CO-CO-; 1,4-dicarbonylphenyl; -$CH_2$-$CH_2$-; or a triazine of formula (b) or (c) below:

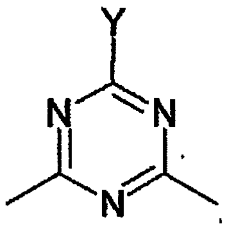

**(b)**

**or**

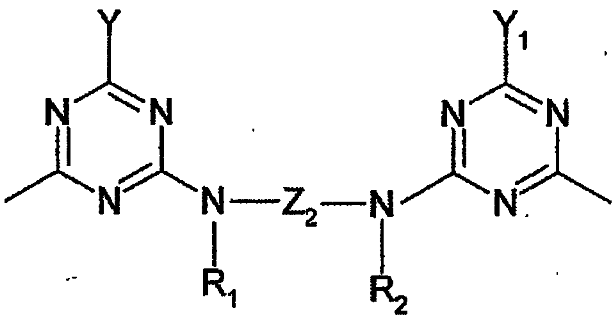

**(c)**

in which:

Y and Y1, independently of each other, denote a halogen atom or a hydroxyl, amino, monoalkylamino, dialkylamino, 1-piperidino, morpholino or 1-piperazino radical, the piperazino radical being unsubstituted or substituted on the nitrogen atom not attached to the triazine ring with a ($C_1$-$C_4$)alkyl radical, said alkyl radicals being unsubstituted or substituted with hydroxyl, amino, mono ($C_1$-$C_4$) alkylamino or di($C_1$-$C_4$)-alkylamino,
- $Z_2$ denotes a ($C_2$-$C_8$)alkylene radical or forms a piperazine ring with the two adjacent nitrogen atoms and the radicals $R_1$ and $R_2$,
- in the radical of formula (a),
- $R_3$ and $R_4$, independently of each other, denote a hydrogen atom or a ($C_1$-$C_4$)alkyl radical, or ($C_1$-$C_4$)alkyl substituted with one or more halogen atoms, a hydroxyl, carboxyl, or cyano radical, a ($C_1$-$C_4$)alkoxy radical, a ($C_1$-$C_4$)alkoxy radical substituted with a hydroxyl or ($C_1$-$C_4$)alkoxy radical, or an amino, alkylamino, dialkylamino, aminocarbonyl, phenyl, phenoxy or phenylaminocarbonyl radical, in which the phenyl radical is unsubstituted or substituted with a ($C_1$-$C_4$)-alkyl, ($C_1$-$C_4$)alkoxy or phenoxy radical,
- $R_5$ and $R_6$, independently of each other, denote a hydrogen atom, a ($C_1$-$C_4$)alkyl or ($C_1$-$C_4$)alkoxy radical optionally substituted with a hydroxyl, carboxyl, halogen or cyano radical, a ($C_1$-$C_4$)alkoxy radical optionally substituted with a hydroxyl or ($C_1$-$C_4$)alkoxy radical, or an amino, alkylamino, dialkylamino, aminocarbonyl, phenyl, phenoxy or phenylaminocarbonyl radical, in which the phenyl radical is unsubstituted or substituted with a ($C_1$-$C_4$)alkyl, ($C_1$-$C_4$)alkoxy or phenoxy radical,
- $An^-$ denotes an anion;

in which formula (III),
- the number of cationic charges is two,
- X' and Y', independently of each other, denote hydrogen, halogen, ($C_1$-$C_4$)alkyl, ($C_1$-$C_4$)alkoxy, ($C_1$-$C_4$)-alkylcarbonylamino, arylcarbonylamino, ureido or arylureido,

- $R'_1$ denotes hydrogen, a substituted alkyl or aryl radical, an unsubstituted alkyl or aryl radical, or has the same meaning as $R'_2$
- $R'_2$ is a radical of formula (d) below:

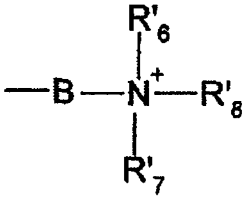

(d)

in which:
- B denotes a linear or branched alkylene radical,
- $R'_6$ denotes hydrogen or substituted or unsubstituted alkyl,
- $R'_7$ and $R'_8$, independently of each other, denote substituted or unsubstituted alkyl,
- $R'_6$ and $R'_7$, together with the nitrogen, form a substituted or unsubstituted 5-, 6- or 7-membered ring, which may contain other hetero atoms, or alternatively $R'_6$, $R'_7$ and $R'_8$ together form a pyridinium ring,
- $R'_3$ denotes hydrogen, halogen, $(C_1-C_4)$alkyl or $(C_1-C_4)$-alkoxy,
- W is a radical of formula (e) below:

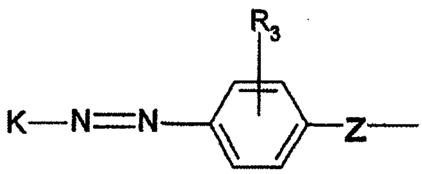

(e)

in which:
- K is a coupling radical,
- Z denotes a bridging radical chosen from the radicals of formulae:

$-NR'_9-CO-$;

$-CO-NR'_9-NR'_9-CO-$;

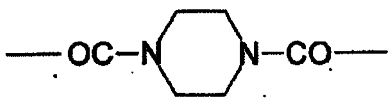

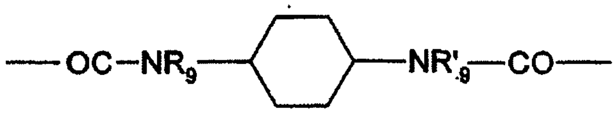

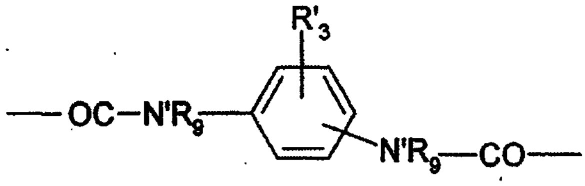

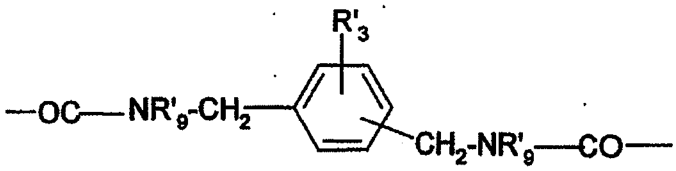

or

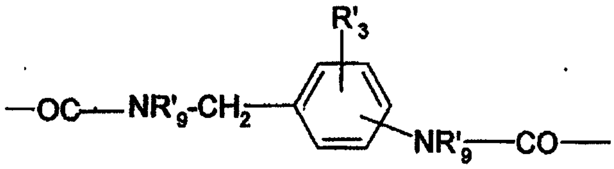

and in which R'$_9$ denotes hydrogen or unsubstituted or substituted ($C_2$-$C_4$)alkylene, the alkylene radical being linear or branched and possibly being interrupted with one or more groups chosen from: -NR'9-, -O- and -S-.

**2.** Use according to Claim 1, **characterized in that**, in formula (I),

- $R_1$ and $R_2$, independently of each other, denote hydrogen, ($C_1$-$C_4$)alkyl substituted with hydroxyl or ($C_1$-$C_4$) alkoxy, and even more particularly denote hydrogen or methyl,
- Z denotes a linear, branched or cyclic $C_2$-$C_8$ alkyl radical optionally substituted with a hydroxyl, alkoxy or halogen, the chain of said radical optionally being interrupted with a group -O- or - $NR_1$-; a 1,4-phenyl radical, a 1,4-naphthyl radical optionally substituted with an alkyl, alkoxy or halogen; Z possibly forming a piperazine ring with $R_1$, $R_2$ and the 2 nitrogen atoms, and preferably Z denotes an unsubstituted phenyl radical, a phenyl or naphthyl radical substituted with 1 or 2 methyl or methoxy radicals, a piperazine radical by bonding with $R_1$, $R_2$ and the 2 nitrogen atoms, or a ($C_2$-$C_4$)alkylene radical which is unsubstituted or substituted with one or two hydroxyls,
- X denotes a group of formula (b).

**3.** Use according to Claim 1, **characterized in that**, in formula (II),

- $Z_1$ denotes a linear, branched or cyclic $C_2$-$C_8$ alkyl radical, optionally substituted with a hydroxyl, alkoxy or halogen, the chain of said radical optionally being interrupted with a group -O- or -$NR_1$-; a piperazine ring formed with $R_1$, $R_2$ and the two nitrogen atoms, and preferably $Z_1$ denotes a ($C_2$-$C_6$)alkylene radical which is unsubstituted or substituted with one or more hydroxyl, a piperazine ring formed with $R_1$, $R_2$ and the two nitrogen atoms; and even more particularly an unsubstituted ($C_2$-$C_4$)alkylene radical,
- $R_3$ and $R_4$ denote methyl or ethyl, and $R_5$ and $R_6$ denote hydrogen, methyl or methoxy.

**4.** Use according to Claim 1, **characterized in that**, in formula (III),
B denotes ethylene, n-propylene, isopropylene or n-butylene,
K denotes a coupling compound chosen from those of formula (f), (g) or (h) below

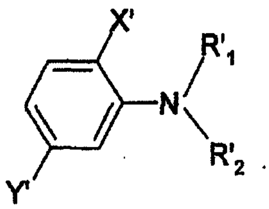

(f)

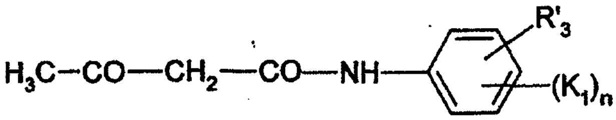

(g)

or

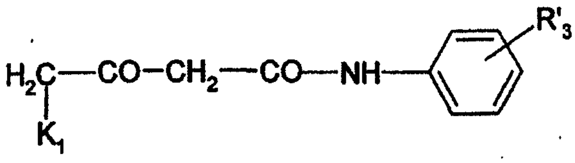

(h)

in which,

- X', Y' and $R'_1$, $R'_2$ and $R'_3$ have the same meaning as in formula (III),
- n is equal to 1 or 2,
- $K_1$ denotes the radical of formula:

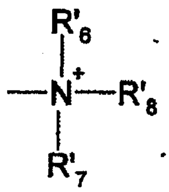

**5.** Use according to either of Claims 1 and 2, **characterized in that** the compound of formula (I) or of formula (II) is chosen from those of formulae (I)1 to (1)7 below:

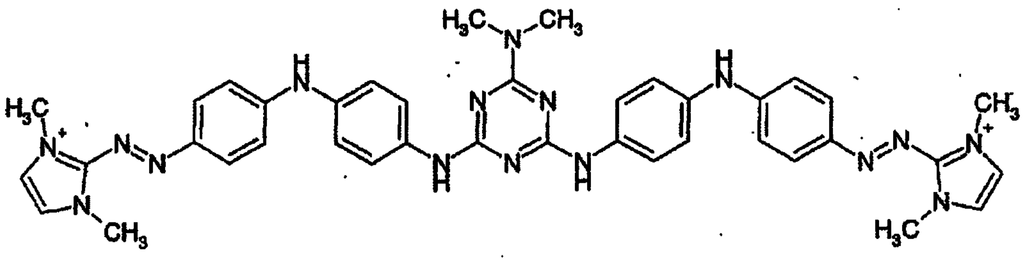

(I)1

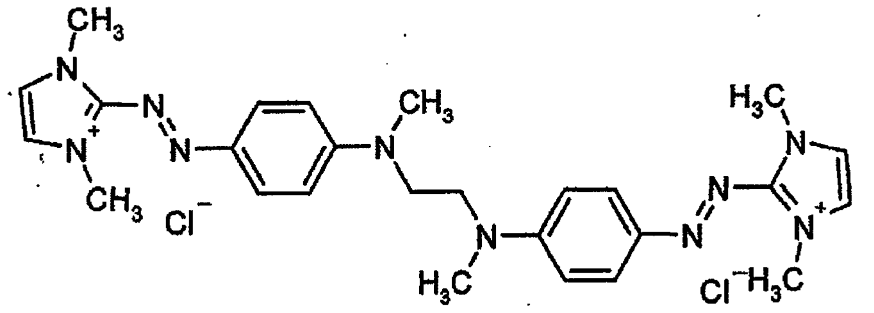

(I)2

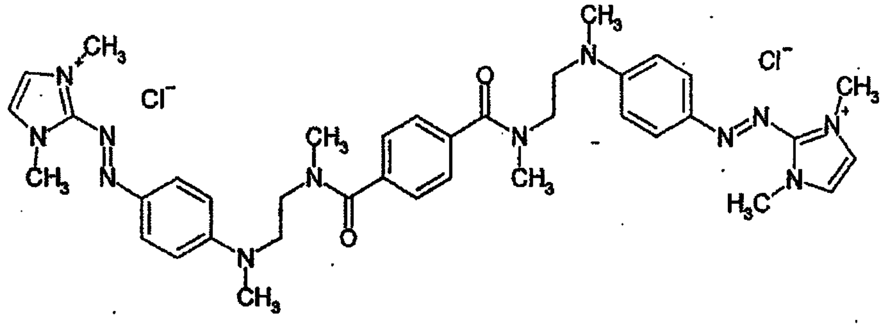

(I)3

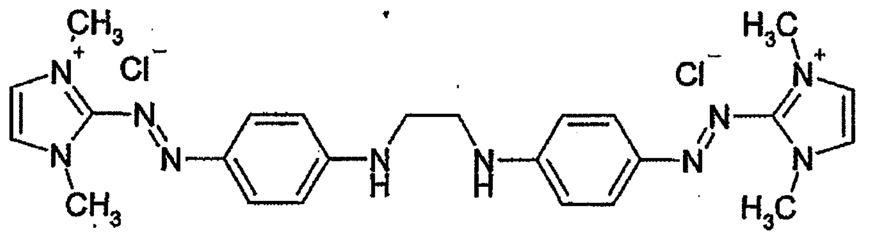

(I)4

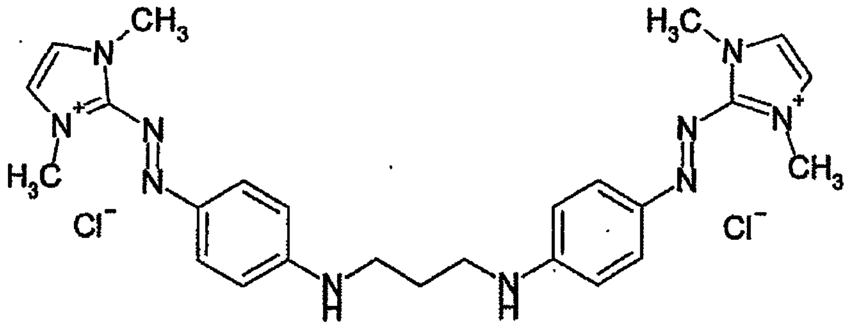

(I)5

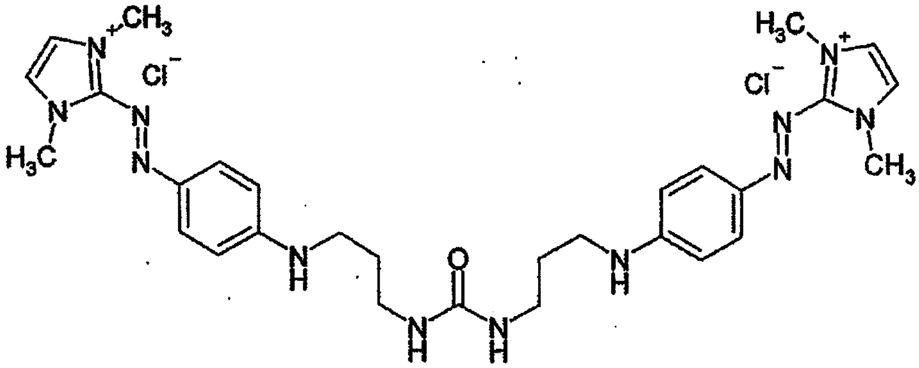

(I)6

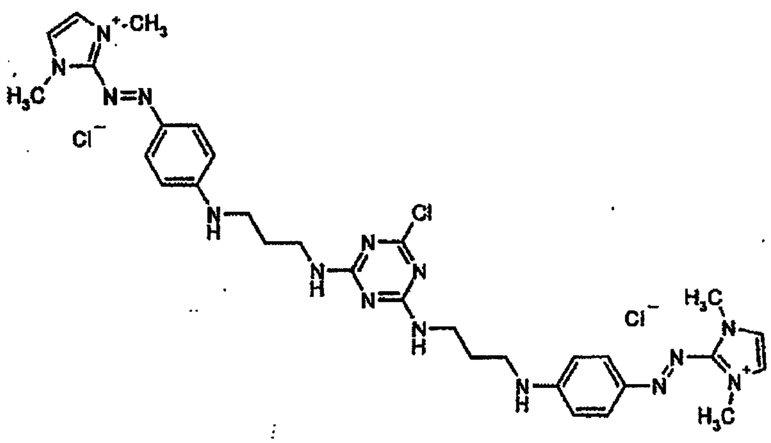

(I)7

6. Use according to either of Claims 1 and 4, **characterized in that** the compound of formula (III) is the following compound:

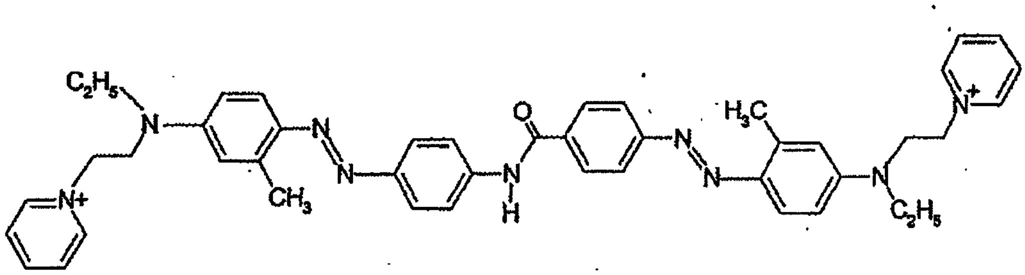

7. Composition for dyeing human keratin fibres, and more particularly the hair, **characterized in that** it comprises an effective amount of at least one direct dye of formula (I), (II) or (III) as defined in any one of the preceding claims, and one or more surfactants chosen without distinction, alone or as mixtures, from anionic, nonionic, amphoteric, zwitterionic and cationic surfactants.

8. Composition according to Claim 7, **characterized in that** the direct dye(s) of formula (I), (II) or (III) is(are) present in an amount ranging from 0.01% to 40% by weight relative to the total weight of the composition.

9. Composition according to Claim 8, **characterized in that** the direct dye(s) of formula (I), (II) or (III) is(are) present in an amount ranging from 0.1% to 20% by weight relative to the total weight of the composition.

10. Composition according to any one of Claims 7 to 9, **characterized in that** it also contains at least one oxidizing agent.

11. Composition according to Claim 10, **characterized in that** the oxidizing agent is chosen from hydrogen peroxide, urea peroxide, alkali metal bromates or ferricyanides, persalts, and redox enzymes, optionally with the respective donor or cofactor thereof.

12. Composition according to Claim 11, **characterized in that** the oxidizing agent is hydrogen peroxide.

13. Composition according to Claim 12, **characterized in that** it is an aqueous hydrogen peroxide solution with a titre ranging from 1 to 40 volumes.

14. Composition according to any one of Claims 7 to 13, **characterized in that** it has a pH of between 2 and 12, preferably between 3 and 11 and more particularly between 7 and 10.

15. Composition according to any one of Claims 7 to 14, **characterized in that** it also contains, in a proportion of from 0.01% to 10% by weight relative to the total weight of the composition, at least one cationic or amphoteric polymer chosen from:

    - 1/ dimethyldiallylammonium chloride homopolymers;
    - 2/ polymers containing repeating units of formula (W) or (U) below:

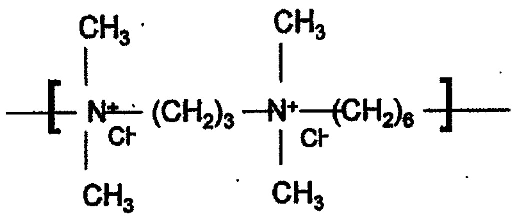

(W)

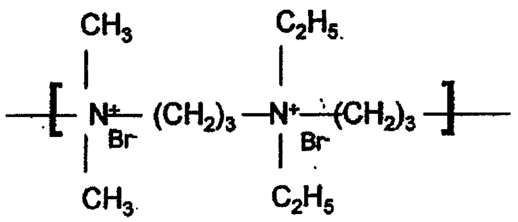

(U)

- 3/ polymers containing units of formula (IX) below:

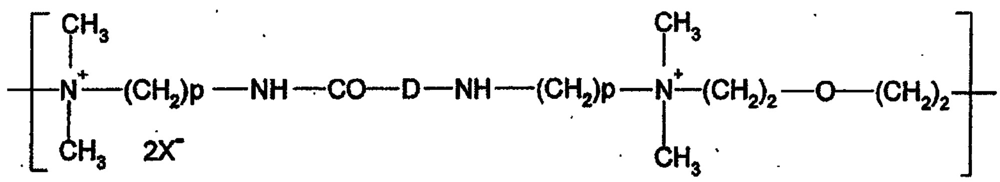

(IX)

for which p is equal to 3, and

a) D denotes the value zero, X denotes a chlorine atom,
b) D represents a $-(CH_2)_4-CO-$ group, X denotes a chlorine atom,
c) D represents a $-(CH_2)_7-CO-$ group, X denotes a chlorine atom,
d) a "Block Copolymer" formed from units corresponding to the polymers described in paragraphs a) and b);

- 4/ copolymers of acrylic acid and of dimethyldiallylammonium chloride.

**16.** Process for dyeing keratin fibres, and in particular the hair, **characterized in that** it consists in applying the dye composition as described in any one of Claims 1 to 6 onto wet or dry fibres, and in leaving it to act for a leave-in time ranging from 5 seconds to 60 minutes, in rinsing the fibres and then optionally in washing them with shampoo, followed by rinsing them again and drying them.

**17.** Process for dyeing keratin fibres, and in particular the hair, **characterized in that** it consists in applying the dye composition as described in any one of Claims 7 to 15 onto wet or dry fibres, and in leaving it to act for a leave-in time ranging from 5 seconds to 60 minutes, in rinsing the fibres and then optionally in washing them with shampoo, followed by rinsing them again and drying them.

**18.** Process according to either of Claims 16 and 17, **characterized in that,** when the composition comprises an oxidizing agent, the oxidizing agent is mixed with the composition just before applying it to the fibres.

**19.** Process according to any one of Claims 16 to 18, **characterized in that** the leave-in time ranges from 10 seconds to 5 minutes and more particularly from 30 seconds to 2 minutes.

**20.** Device for the lightening direct dyeing of human keratin fibres, and in particular the hair, **characterized in that** it comprises a first compartment containing, in a medium that is suitable for dyeing, at least one direct dye of formula (I), (II) or (III), which formulae are defined in any one of Claims 1 to 6, and a second compartment containing an oxidizing agent.

**Patentansprüche**

**1.** Verwendung von Verbindungen der nachstehenden Formeln (I), (II) oder (III) zum Färben von menschlichen Keratinfasern und insbesondere der Haare als Direktfarbstoffe in einer kosmetischen Zusammensetzung,

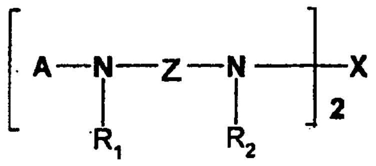

(I),

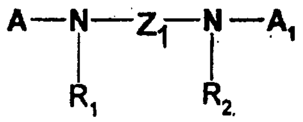

(II),

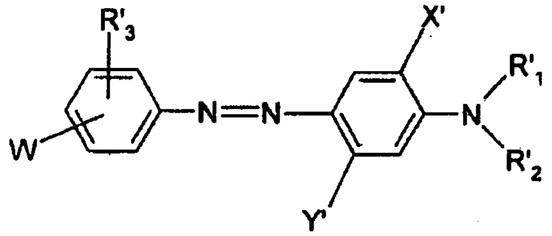

(III),

wobei in den **Formeln (I) oder (II)** bedeuten:

- A und $A_1$ unabhängig voneinander eine Gruppe der nachstehenden Formel (a)

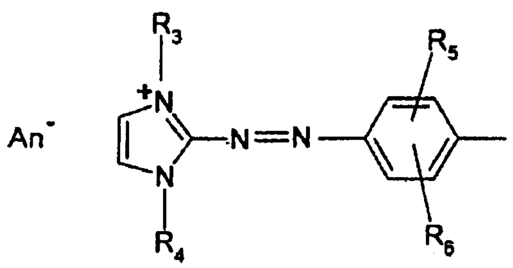

(a),

- Z eine aliphatische oder aromatische Gruppe,
- $Z_1$ eine Alkylgruppe,
- $R_1$ und $R_2$ unabhängig voneinander ein Wasserstoffatom oder $C_1$-$C_4$-Alkyl oder $C_1$-$C_4$-Alkyl, das mit einem oder mehreren Halogenatomen substituiert ist, Hydroxy, Carboxy, Cyano, $C_1$-$C_4$-Alkoxy, $C_1$-$C_4$-Alkoxy, das mit einer oder mehreren Hydroxygruppen oder $C_1$-$C_4$-Alkoxy substituiert ist, Amino, Alkylamino, Dialkylamino, Aminocarbonyl, Phenyl, Phenoxy oder Phenylaminocarbonyl, worin die Phenylgruppe unsubstituiert oder mit einer $C_1$-$C_4$-Alkylgruppe, $C_1$-$C_4$-Alkoxygruppe oder Phenoxy substituiert ist, oder $R_1$ und $R_2$ bilden zusammen mit den beiden Stickstoffatomen, die sie tragen, und der Gruppe Z einen Piperazinring,
- X eine Brückengruppe, die ausgewählt ist unter: -CO-; $-CO-CH_2-CH_2-CO$; -CO-CO-, 1,4-Dicarbonylphenyl; $-CH_2-CH_2-$oder einem Triazin der nachstehenden Formeln (b) oder (c),

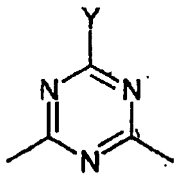

(b)

oder

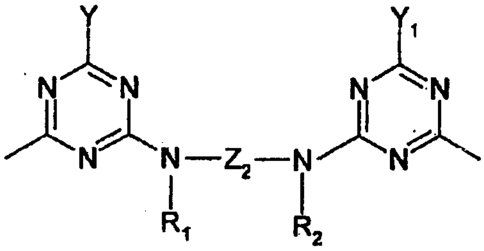

(c),

wobei in diesen Formeln bedeuten:
Y und $Y_1$ unabhängig voneinander ein Halogenatom oder eine Hydroxygruppe oder Amino oder Monoalkylamino oder
Dialkylamino oder 1-Piperidino oder Morpholino oder 1-Piperazino, wobei die Piperazino-Gruppe unsubstituiert oder an dem nicht am Triazinring gebundenen Stickstoffatom mit einer $C_1$-$C_4$-Alkylgruppe substituiert ist, wobei die Alkylgruppen unsubstituiert oder durch Hydroxy, Amino, Mono-($C_1$-$C_4$)-alkylamino oder Di-($C_1$-$C_4$)-alkylamino substituiert sind,
$Z_2$ $C_2$-$C_8$-Alkylen oder bildet mit den beiden benachbarten Stickstoffatomen und den Gruppen $R_1$ und $R_2$ einen Piperazinring,
- wobei in der Gruppe der Formel (a) bedeuten:

- $R_3$ und $R_4$ unabhängig voneinander ein Wasserstoffatom oder $C_1$-$C_4$-Alkyl oder $C_1$-$C_4$-Alkyl, das mit einem oder mehreren Halogenatomen substituiert ist, eine Hydroxygruppe, Carboxy, Cyano, $C_1$-$C_4$-Alkoxy, $C_1$-$C_4$-Alkoxy, das mit einer Hydroxygruppe oder $C_1$-$C_4$-Alkoxy substituiert ist, Amino, Alkylamino, Dialkylamino, Aminocarbonyl, Phenyl, Phenoxy oder Phenylaminocarbonyl, worin die Phenylgruppe unsubstituiert oder mit einer $C_1$-$C_4$-Alkylgruppe, einer $C_1$-$C_4$-Alkoxygruppe oder Phenoxy substituiert ist,
- $R_5$ und $R_6$ unabhängig voneinander ein Wasserstoffatom, $C_1$-$C_4$-Alkyl oder $C_1$-$C_4$-Alkoxy, die gegebenenfalls mit einer Hydroxygruppe, Carboxy, Halogen, Cyano substituiert sind, $C_1$-$C_4$-Alkoxy, das gegebenenfalls mit einer Hydroxygruppe oder einer $C_1$-$C_4$-Alkoxygruppe substituiert ist, Amino, Alkylamino, Dialkylamino, Aminocarbonyl, Phenyl, Phenoxy oder Phenylaminocarbonyl, worin die Phenylgruppe unsubstituiert oder mit einer $C_1$-$C_4$-Alkylgruppe, einer $C_1$-$C_4$-Alkoxygruppe oder Phenoxy substituiert ist.

- An- ein Anion,
und wobei in **Formel (III)**
- die Anzahl der kationischen Ladungen gleich 2 ist, und bedeuten:

- X' und Y' unabhängig voneinander Wasserstoff, Halogen, $C_1$-$C_4$-Alkyl, $C_1$-$C_4$-Alkoxy, $C_1$-$C_4$-Alkylcarbonylamino, Arylcarbonylamino, Ureido oder Arylureido,
- $R'_1$ Wasserstoff, eine Alkylgruppe oder eine Arylgruppe, die substituiert sind, eine Alkylgruppe oder eine Arylgruppe, die nicht substituiert sind, oder dasselbe wie $R'_2$,
- $R'_2$ eine Gruppe der nachstehenden Formel (d):

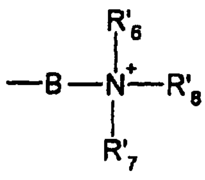

(d);

worin bedeuten:

- B eine geradkettige oder verzweigte Alkylengruppe,
- $R'_6$ Wasserstoff oder substituiertes oder unsubstituiertes Alkyl,
- $R'_7$ und $R'_8$ unabhängig voneinander substituiertes oder unsubstituiertes Alkyl,

oder

- $R'_6$ und $R'_7$ bilden zusammen mit dem Stickstoff einen fünfgliedrigen, sechsgliedrigen oder siebengliedrigen Ring, der substituiert oder unsubstituiert ist und weitere Heteroatome enthalten kann,

oder

- $R'_6$ und $R'_7$ und $R'_8$ bilden zusammen eine Pyridiniumring,

- $R'_3$ Wasserstoff, Halogen, $C_1$-$C_4$-Alkyl, $C_1$-$C_4$-Alkoxy,
- W eine Gruppe der nachstehenden Formel (e)

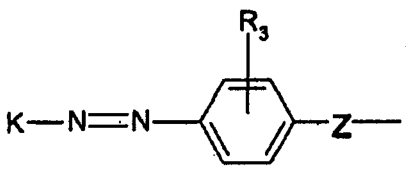

(e),

worin bedeuten:

- K eine Kupplungsgruppe,
- Z eine Brückengruppe, die ausgewählt ist unter den Gruppen der Formeln:

-NR'$_9$-CO- ,

-CO-NR'$_9$-NR'$_9$-CO- ,

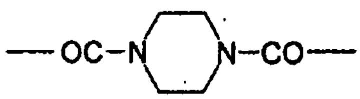

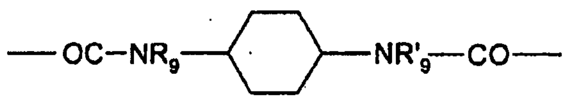

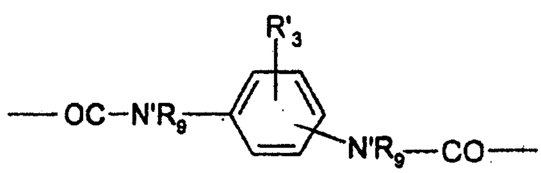

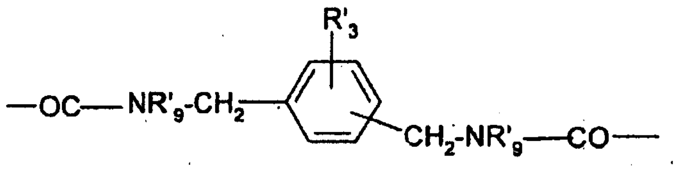

oder

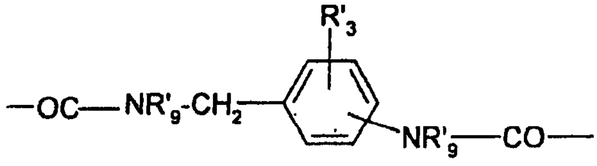

,

und wobei in diesen Formeln R'$_9$ bedeutet: Wasserstoff, $C_2$-$C_4$-Alkylen, das unsubstituiert oder substituiert ist, wobei die Alkylengruppe geradkettig oder verzweigt ist und durch ein oder mehrere Elemente unterbrochen sein kann, die ausgewählt sind unter: -NR'$_9$-, -O-, -S-.

**2.** Verwendung nach Anspruch 1, **dadurch gekennzeichnet, dass** in Formel (I) bedeuten:

- $R_1$ und $R_2$ unabhängig voneinander Wasserstoff, $C_1$-$C_4$-Alkyl, das mit Hydroxy oder $C_1$-$C_4$-Alkoxy substituiert ist, und insbesondere Wasserstoff oder Methyl,
- Z eine geradkettige oder verzweigte oder cyclische $C_2$-$C_8$-Alkylgruppe, die gegebenenfalls mit einer Hydroxygruppe, einer Alkoxygruppe oder Halogen substituiert ist, wobei die Kette dieser Gruppe gegebenenfalls durch -O- oder -$NR_1$-unterbrochen ist; eine 1,4-Phenyl-Gruppe, eine 1,4-NaphthylGruppe, die gegebenenfalls mit einer Alkylgruppe, einer Alkoxygruppe oder Halogen substituiert ist; oder Z bildet mit $R_1$, $R_2$ und den 2 Stickstoffatomen einen Piperazinring, und vorzugsweise bezeichnet Z eine unsubstituierte Phenylgruppe, eine Phenylgruppe oder Naphthylgruppe, die mit 1 oder 2 Methylgruppen oder Methoxygruppen substituiert ist, eine Piperazingruppe durch Bindung mit $R_1$, $R_2$ und den zwei Stickstoffatomen, eine $C_2$-$C_4$-Alkylengruppe, die unsubstituiert oder mit einer oder zwei Hydroxygruppen substituiert ist,
- X eine Gruppe der Formel (b).

**3.** Verwendung nach Anspruch 1, **dadurch gekennzeichnet, dass** in Formel (II) bedeuten:

- Z1 eine geradkettige oder verzweigte oder cyclische $C_2$-$C_8$-Alkylgruppe, die gegebenenfalls mit einer Hydroxygruppe, einer Alkoxygruppe oder Halogen substituiert ist, wobei die Kette dieser Gruppe gegebenenfalls durch -O- oder -$NR_1$-unterbrochen ist; einen Piperazinring, der mit $R_1$, $R_2$ und den beiden Stickstoffatomen gebildet ist, und vorzugsweise eine $C_2$-$C_6$-Alkylengruppe, die unsubstituiert oder mit einer oder mehreren Hydroxygruppen substituiert ist, einen Piperazinring, der mit $R_1$, $R_2$ und den beiden Stickstoffatomen gebildet ist, und ganz besonders eine unsubstituierte $C_2$-$C_4$-Alkylengruppe,
- $R_3$ und $R_4$ Methyl oder Ethyl und
- $R_5$ und $R_6$ Wasserstoff, Methyl oder Methoxy.

**4.** Verwendung nach Anspruch 1, **dadurch gekennzeichnet, dass** in Formel (I) bedeuten:

- B Ethylen, n-Propylen, Isopropylen oder n-Butylen,
- K eine Kupplerverbindung, die unter den Verbindungen der nachstehenden Formeln (f), (g) oder (h) ausgewählt ist,

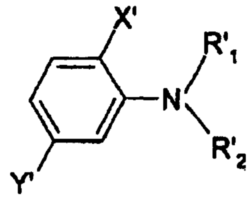

(f),

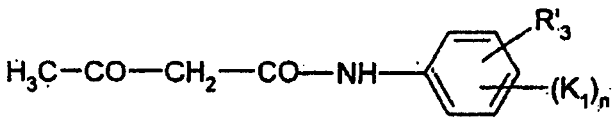

(g)

oder

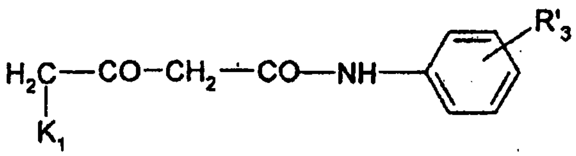

(h),

worin bedeuten:

- X', Y' und $R'_1$, $R'_2$ und $R'_3$ dasselbe wie in Formel (III),
- n 1 oder 2,
- $K_1$ die Gruppe der Formel

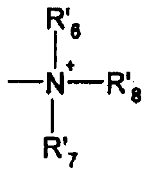

.

**5.** Verwendung nach Anspruch 1 oder 2, **dadurch gekennzeichnet, dass** die Verbindung der Formel (I) oder der Formel (II) unter den Verbindungen der nachstehenden Formeln (I) 1 bis (1)7 ausgewählt ist:

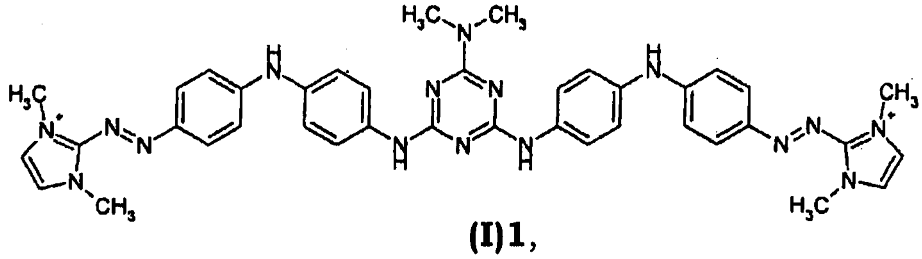

(I)1,

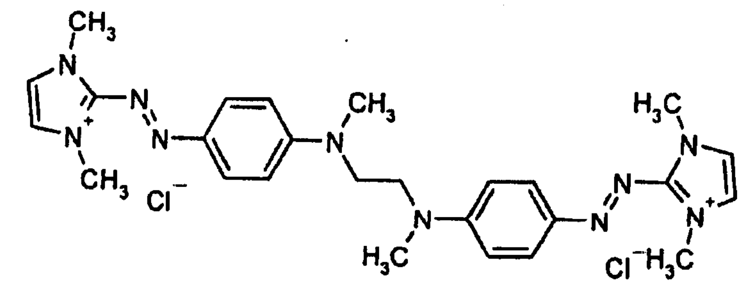

(I)2,

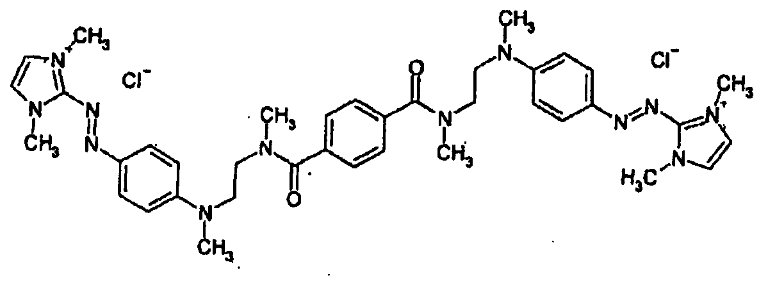

(I)3,

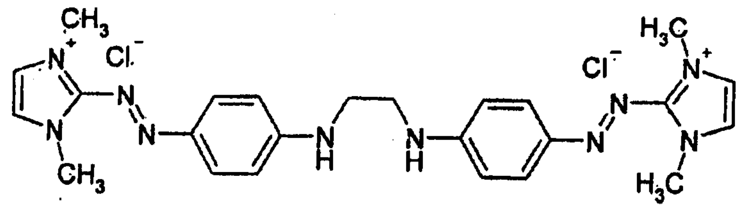

(I)4,

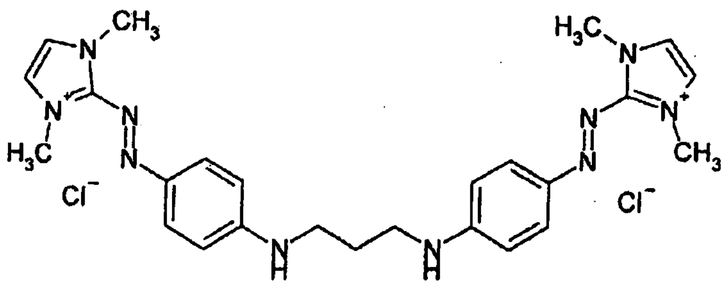

(I)5,

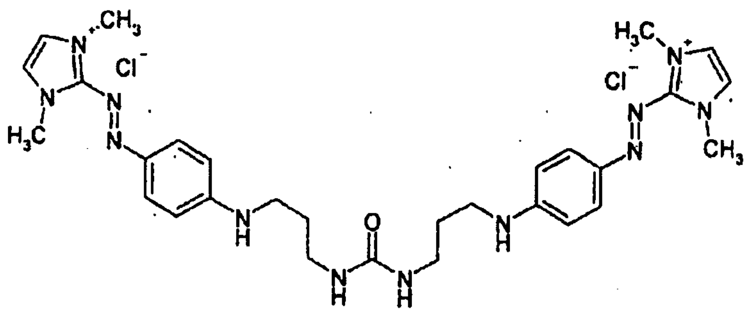

(I)6,

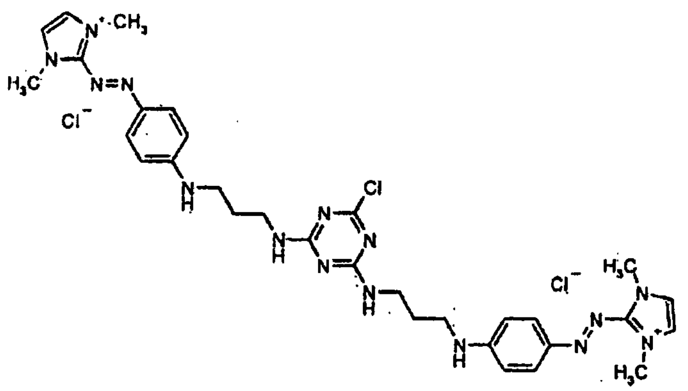

(I)7.

6. Verwendung nach Anspruch 1 oder 4, **dadurch gekennzeichnet, dass** es sich bei der Verbindung der Formel (III) um folgende Verbindung handelt:

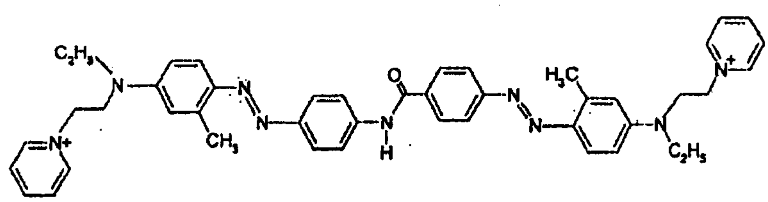

7. Zusammensetzung zum Färben von menschlichen Keratinfasern und insbesondere der Haare, **dadurch gekennzeichnet, dass** sie eine wirksame Menge mindestens eines Direktfarbstoffs der Formeln (I), (II) oder (III), wie sie in einem der vorhergehenden Ansprüche definiert sind, und ein oder mehrere Tenside enthält, die allein oder in Form von Gemischen unter den anionischen, nichtionischen, amphoteren, zwitterionischen und kationischen Tensiden ausgewählt sind.

8. Zusammensetzung nach Anspruch 7, **dadurch gekennzeichnet, dass** der Direktfarbstoff oder die Direktfarbstoffe der Formeln (I), (II) oder (III) in einer Menge von 0,01 bis 40 Gew.-% vorliegen, bezogen auf das Gesamtgewicht der Zusammensetzung.

9. Zusammensetzung nach Anspruch 8, **dadurch gekennzeichnet, dass** der Direktfarbstoff oder die Direktfarbstoffe der Formel (I), (II) oder (III) in einer Menge von 0,1 bis 20 Gew.-% vorliegen, bezogen auf das Gesamtgewicht der Zusammensetzung.

10. Zusammensetzung nach einem der Ansprüche 7 bis 9, **dadurch gekennzeichnet, dass** sie ferner mindestens ein Oxidationsmittel enthält.

11. Zusammensetzung nach Anspruch 10, **dadurch gekennzeichnet, dass** das Oxidationsmittel ausgewählt ist unter Wasserstoffperoxid, Harnstoffperoxid, Alkalibromaten oder Alkalihexacyanoferrat(III)en, Persalzen und Oxidoreductasen, gegebenenfalls mit ihrem entsprechenden Donor oder Cofaktor.

12. Zusammensetzung nach Anspruch 11, **dadurch gekennzeichnet, dass** das Oxidationsmittel Wasserstoffperoxid ist.

13. Zusammensetzung nach Anspruch 12, **dadurch gekennzeichnet, dass** es sich um eine Lösung von Wasserstoffperoxid mit einem Titer von 1 bis 40 Volumina handelt.

14. Zusammensetzung nach einem der Ansprüche 7 bis 13, **dadurch gekennzeichnet, dass** sie einen pH-Wert von 2 bis 12, vorzugsweise von 3 bis 11 und ganz besonders von 7 bis 10 besitzt.

15. Zusammensetzung nach einem der Ansprüche 7 bis 14, **dadurch gekennzeichnet, dass** sie ferner in einem Mengenanteil von 0,01 bis 10 Gew.-%, bezogen auf das Gesamtgewicht der Zusammensetzung, ein kationisches oder amphoteres Polymer enthält, das ausgewählt ist unter

1) Homopolymeren von Dimethyldiallylammoniumchlorid;
2) Polymeren mit wiederkehrenden Einheiten der nachstehenden Formeln (W) oder (U):

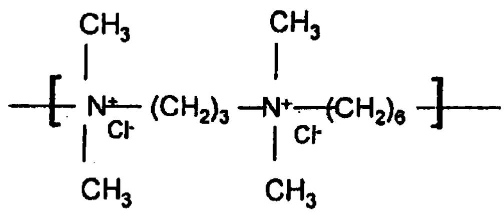

**(W),**

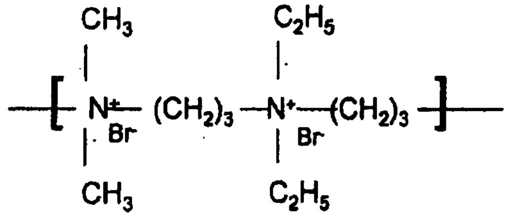

**(U),**

3) Polymeren mit Einheiten der nachstehenden Formel (IX):

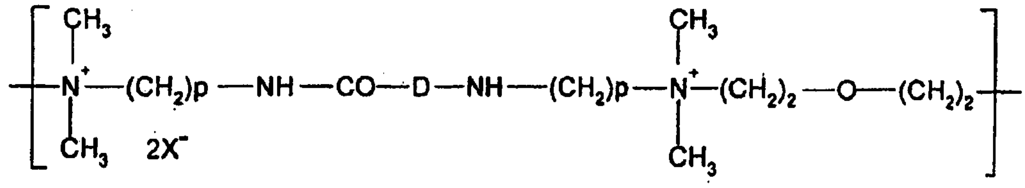

**(IX),**

worin p gleich 3 ist und

a) D den Wert Null und X ein Chloratom bezeichnen,
b) D eine Gruppe $-(CH_2)_4-CO-$ und X ein Chloratom bezeichnen,
c) D eine Gruppe $-(CH_2)_7-CO-$ und X ein Chloratom bezeichnen,
d) es sich um ein "Blockcopolymer" handelt, das aus Einheiten aufgebaut ist, die den in den Absätzen a) und b) beschriebenen Polymeren entsprechen;

4) Copolymeren von Acrylsäure und Dimethyldiallylammoniumchlorid.

**16.** Verfahren zum Färben von Keratinfasern und insbesondere der Haare, **dadurch gekennzeichnet, dass** es darin besteht, die Färbezusammensetzung, wie sie in einem der Ansprüche 1 bis 6 beschrieben ist, auf die trockenen oder feuchten Fasern aufzubringen und sie während einer Ruhezeit von 5 Sekunden bis 60 Minuten einwirken zu lassen, die Fasern zu spülen, sie gegebenenfalls anschließend mit Shampoo zu waschen, sie dann wiederum zu spülen und dann zu trocknen.

**17.** Verfahren zum Färben von Keratinfasern und insbesondere der Haare, **dadurch gekennzeichnet, dass** es darin besteht, die Färbezusammensetzung, wie sie in einem der Ansprüche 7 bis 15 beschrieben ist, auf die trockenen oder feuchten Fasern aufzubringen und sie während einer Ruhezeit von 5 Sekunden bis 60 Minuten einwirken zu lassen, die Fasern zu spülen, sie gegebenenfalls anschließend mit Shampoo zu waschen, sie dann wiederum zu spülen und dann zu trocknen.

**18.** Verfahren nach Anspruch 16 oder 17, **dadurch gekennzeichnet, dass,** wenn die Zusammensetzung ein Oxidationsmittel enthält, das Oxidationsmittel unmittelbar vor dem Aufbringen auf die Fasern zugemischt wird.

**19.** Verfahren nach einem der Ansprüche 16 bis 18, **dadurch gekennzeichnet, dass** die Ruhezeit im Bereich von 10 Sekunden bis 5 Minuten und insbesondere im Bereich von 30 Sekunden bis 2 Minuten liegt.

**20.** Vorrichtung zur aufhellenden Direktfärbung von menschlichen Keratinfasern und insbesondere der Haare, **dadurch gekennzeichnet, dass** sie aufweist: ein erstes Compartment, das in einem zum Färben geeigneten Medium mindestens einen Direktfarbstoff der Formeln (I), (II) oder (III), wie sie in einem der Ansprüche 1 bis 6 definiert sind, enthält, sowie ein zweites Compartment, das ein Oxidationsmittel enthält.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2586913
- US 5674299 A
- US 5708151 A
- EP 0216479 A
- US 3915921 A
- US 4509949 A
- EP 0173109 A
- WO 9844012 A
- EP 337354 A
- FR 2270846
- FR 2383660
- FR 2598611
- FR 2470596
- FR 2519863
- FR 2505348
- FR 2542997
- EP 080976 A
- FR 2077143
- FR 2393573
- FR 2162025
- FR 2280361
- FR 2252840
- FR 2368508
- FR 1583363
- FR 2080759
- FR 2190406
- FR 2320330
- FR 2316271
- FR 2336434
- FR 2413907
- US 2273780 A
- US 2375853 A
- US 2388614 A
- US 2454547 A
- US 3206462 A
- US 2261002 A
- US 2271378 A
- US 3874870 A
- US 4001432 A
- US 3929990 A
- US 3966904 A
- US 4005193 A
- US 4025617 A
- US 4025627 A
- US 4025653 A
- US 4026945 A
- US 4027020 A
- EP 122324 A
- US 4157388 A
- US 4390689 A
- US 4702906 A
- US 4719282 A
- FR 2137684
- US 3879376 A
- FR 1400366
- US 2528378 A
- US 2781354 A


### Littérature non-brevet citée dans la description


- **G. Fonnum ; J. Bakke ; Fk. Hansen.** *Colloid Polym. Sci,* 1993, vol. 271, 380.389
- **M.R. PORTER.** Handbook of Surfactants. éditions Blackie & Son, 1991, 116-178